# EUROPEAN PATENT SPECIFICATION

(11) **EP 3 775 162 B1**
(45) Date of publication and mention of the grant of the patent: **06.08.2025**
(21) Application number: 19812157.6
(22) Date of filing: 31.05.2019
(51) Int. Cl.: C12N 5/071, A61K 35/36, C12Q 1/70, C12R 1/93, C12N 5/00, G01N 33/50

(54) **MATURE AIRWAY ORGANOIDS, METHODS OF MAKING AND USES THEREOF**
REIFE ATEMWEGSORGANOIDE, VERFAHREN ZU IHRER HERSTELLUNG UND VERWENDUNGEN
ORGANOÏDES DE VOIES AÉRIENNES MATURES, LEURS PROCÉDÉS DE FABRICATION ET LEURS UTILISATIONS

(30) Priority: 02.06.2018 US 201862679788 P
(43) Date of publication of application: 17.02.2021
(73) Proprietor: The University of Hong Kong, Hong Kong (HK); Koninklijke Nederlandse Akademie van Wetenschappen, 1011 JV Amsterdam (NL)
(72) Inventor: ZHOU, Jie, Hong Kong (HK); YUEN, Kwok Yung, Hong Kong (HK); LI, Cun, Hong Kong (HK); CHIU, Man Chun, Hong Kong (HK); CLEVERS, Johannes Carolus, 1015AA Amsterdam (NL)
(74) Representative: HGF
(86) International application number: PCT/CN2019/089613
(87) International publication number: WO 2019/228516

(56) References cited:
- WO-A1-2015/108893
- WO-A1-2017/066507
- WO-A2-2018/129507
- GB-A- 2 532 814
- US-A1- 2009 227 025
- QUANTIUS JENNIFER ET AL: "Influenza Virus Infects Epithelial Stem/Progenitor Cells of the Distal Lung: Impact on Fgfr2b-Driven Epithelial Repair", PLOS PATHOGENS, vol. 12, no. 6, 20 June 2016 (2016-06-20), pages e1005544, XP055864997, Retrieved from the Internet <URL:https://journals.plos.org/plospathogens/article/file?id=10.1371/journal.ppat.1005544&type=printable> DOI: 10.1371/journal.ppat.1005544
- YAMAMOTO YUKI ET AL: "Long-term expansion of alveolar stem cells derived from human iPS cells in organoids", NATURE METHODS, vol. 14, no. 11, 2 October 2017 (2017-10-02), New York, pages 1097 - 1106, XP055865567, ISSN: 1548-7091, Retrieved from the Internet <URL:https://www.nature.com/articles/nmeth.4448.pdf> DOI: 10.1038/nmeth.4448
- ZHOU JIE ET AL: "Differentiated human airway organoids to assess infectivity of emerging influenza virus", PROCEEDINGS OF THE NATIONAL ACADEMY OF SCIENCES, vol. 115, no. 26, 26 June 2018 (2018-06-26), pages 6822 - 6827, XP055864991, ISSN: 0027-8424, Retrieved from the Internet <URL:https://www.pnas.org/content/pnas/115/26/6822.full.pdf> DOI: 10.1073/pnas.1806308115
- YUEN ET AL: "Establishing Mature Airway Organoid to Assess Infectivity of Emerging Influenza Virus", 1 June 2018 (2018-06-01), XP055865001, Retrieved from the Internet <URL:https://www.med.hku.hk/f/news/2570/5285/Mature%20human%20airway%20organoid_PPT.pdf> [retrieved on 20211123]
- TAN Q . ET AL.: "Human airway organoid engineering as a step toward lung regeneration and disease modeling", BIOMATERIALS, vol. 113, 28 October 2016 (2016-10-28), pages 118 - 132, XP029812737, ISSN: 0142-9612, DOI: 10.1016/j.biomaterials.2016.10.046
- DYE B.R. ET AL.: "Take a deep breath and digest the material:organoids and biomaterials of the respiratory and digestive systems", MRS COMMUNICATIONS, vol. 7, no. 3, 1 September 2017 (2017-09-01), pages 502 - 514, XP055659161, ISSN: 2159-6859, DOI: 10.1557/mrc.2017.61

## Description

### FIEED OF THE INVENTION

The invention is generally directed to airway organoids, particularly differentiated airway organoids, methods of making and using, particularly for influenza virus research.

### BACKGROUND OF THE INVENTION

Influenza A viruses (IAVs) can infect a diversity of avian and mammalian species including humans, and have the remarkable capacity to evolve and adapt to new hosts (1). The segmented RNA genomes of IAVs and the low fidelity of RNA polymerase allow for antigenic shift and drift, which drive this evolution. Thus, novel viruses from birds and pigs can cross the species barrier and infect humans, leading to sporadic infections, epidemics and even pandemics (Klenk, Cell Host Microbe 15(6):653-654 (2014); To, et al., Lancet 381 (9881): 1916-1925 (2013)). Despite the tremendous progress made in virology and epidemiology, it remains unpredictable which subtype or strain of IAV will cause the next outbreak. A novel reassortant H7N9 influenza virus from poultry has led to recurrent outbreaks of human infections in China since 2013 (To, et al, Lancet 381(9881): 1916-1925 (2013)), Chen, et al., Lancet 381(9881): 1916-1925 (2013)). According to a World Organization report more than 1500 laboratory-confirmed cases of H7N9 human infections were reported by October 2017, with a case-fatality rate higher than 35%. In 2009, the first influenza pandemic of the 2 1^{sₜ} century was caused by a novel pandemic H1N1 (HlNlpdm), which originated via multiple reassortment of "classical" swine H1N1 virus with human H3N2 virus, avian virus and avian-like swine virus (AVIT, et al, N Engl J Med 360(25):2605-2615 (2009)). While swine viruses only sporadically infect humans, this novel strain of swine-derived HlNlpdm vims can establish sustained human-to-human transmission and has been circulating globally as a seasonal virus strain since then. Proteolytic cleavage of viral glycoprotein hemagglutinin (HA) is essential for IAV to acquire infectivity since only the cleaved HA molecule mediates the membrane fusion between vims and host cell, a process required for the initiation of infection. HA proteins of low pathogenic avian IAVs and human IAVs carry a single basic amino acid arginine at the cleavage site (Bottcher E, et al, J Virol 80(1 9): 9896-9898 (2006); Bosch, et al, Virology 113(2):725-735 (1981)), recognized by trypsin-like serine proteases. Productive infection of these viruses in human airway thus requires serine proteases like TMPRSS2, TMPRSS4, HAT etc. (Bottcher-Friebertshauser et al., Pathog Dis 69(2):87-100 (2013). However, HA proteins of high pathogenic avian viruses, such as H5N1, contain a polybasic cleavage site that is activated by ubiquitously expressed proteases.

Current *in vitro* models for studying influenza infection in human respiratory tract involve short-term cultures of human lung explant and primary airway epithelial cells. Human lung explants are not readily available on a routine basis. In addition, rapid deterioration of primary tissue in infection experiments is a major problem. Under air-liquid interface conditions, basal cells isolated from human airway can polarize and undergo mucociliary differentiation. Yet, this capacity is lost within 2-3 passages (Butler, et al., Am J Respir Crit Care Med 194(2): 156-168 (2016)). Collectively, these primary tissues and cells barely constitute a convenient, reproducible model to study human respiratory pathogens. Although various cell lines, e.g. A549 and MDCK, have commonly been used to propagate influenza viruses and to study virology, they poorly recapitulate the histology of human airway epithelium. In addition, due to the low serine protease activity, most cell lines do not support the growth of the influenza viruses with monobasic HA cleavage site unless the culture medium is supplemented the exogenous serine protease, trypsin treated with N-tosyl-L-phenylalanine chloromethyl ketone (TPCK) . Thus, a biologically-relevant, reproducible, and readily-available *in vitro* model remains desperately needed for studying biology and pathology of the human respiratory tract.

Recent advances in stem cell biology have allowed the *in vitro* growth of 3 dimensional (3D) organoids that recapitulate essential attributes of their counterpart-organs *in vivo.* These organoids can be grown from pluripotent stem cells (PSC) or tissue-resident adult stem cells (ASC) (Clevers, et al, Cell 165(7): 1586-1597 (2016)). ASC-derived organoids consist exclusively of epithelial cells and can be generated from a variety of human organs, the first being the human gut (Sato, et al., Gastroenterology 141(5): 1762-1772 (2011)). These human intestinal organoids represent the first model for *in vitro* propagation of Norovirus and has allowed the study of other viruses (Ettayebi, et al., Science, 353:1387-1393 (2016); Zhou, et al., Sci. Adv. 3(ll);eaao4966 (2017)). ASC-derived lung organoids have also been described (WO201 6/08361 3).

Of note, protocols have also been established to generate lung organoids from human PSCs, embryonic lung (Chen, et al., Nat Cell Biol 19(5): 542-549, (2017); Nikolic, et al., Elife 6: e26575 (2017)), embryonic stem cells and induced pluripotent stem cells (iPSC) (Konishi, et al, Stem Cell Reports, 6(1): 18-25 (2016)).

However, there is still a need for improved methods of generating in vitro cellular systems that recapitulate the histology and functionality of mature (differentiated) human airway epithelium, for example, for use in modelling infection, particularly influenza infection. There is, in particular, a need for improved methods of differentiating ASC-derived lung organoids. Such methods would be advantageous because they do not rely on induced pluripotent stem cells, embryonic stem cells or embryonic lung. Therefore, it is the object of the present invention to provide a method of generating an in vitro cellular system that recapitulates the histology and functionality of mature human airway epithelium for use in modelling diseases, for example, influenza infection.

It is another object of the present invention to provide a method of differentiating lung organoids, preferably wherein said method does not rely on induced pluripotent stem cells, embryonic stem cells or embryonic lung.

It is another object of the present invention to provide improved *in vitro* differentiated lung organoids that recapitulate the histology of human airway epithelium.

It is yet another object of the present invention to provide methods for studying the biology and pathology of the human airway epithelium.
TAN Q. et al.: "Human airway organoid engineering as a step toward lung regeneration and disease modeling", Biomaterials, vol. 113, 28 October 2016 (2016-10-28), pages 118-132, a method to obtain a human proximal differentiated airway organoid, that express the ciliated cell marker FOXJ1. The organoid is obtained by cultivating NHBE cells undergoing differentiation in 3D airway organoid culture with DAPT at 2µM.

### SUMMARY OF THE INVENTION

In a first aspect of the invention is provided, a method of generating a proximal differentiated airway organoid (PD-organoid) comprising culturing an airway organoid (AO-organoid) derived from adult stem cells (ASC) in a proximal differentiation medium for a period of time sufficient to generate a PD-organoid comprising a cell population consisting of at least 25%, at least 30%, at least 35% or at least 40% ciliated cells, wherein the ciliated cells are characterised by FOXJ1 and SNTN expression;
wherein the method further comprises one or more of the following steps prior to culturing the AO-organoid in a proximal differentiation medium:
   a. obtaining a lung tissue sample from a subject;
   b. obtaining dissociated cells from a lung tissue sample; and
   c. culturing lung cells in an AO-organoid formation phase for a period of time sufficient to generate an AO-organoid; and
wherein the proximal differentiation medium is supplemented with DAPT at a concentration of between 10 and 20 µM.

In some embodiments, the proximal differentiation medium comprises one or more components as set out in Table 2, optionally at the concentrations shown in Table 2.

In some embodiments, the proximal differentiation medium comprises at least EGF, insulin, transferrin, hydrocortisone, triiodothyronine and epinephrine.

In some embodiments, the proximal differentiation medium further comprises bovine serum albumin and/or bovine pituitary extract.

In some embodiments, the proximal differentiation medium is PneumaCult-ALI medium (StemCell Technologies).

In some embodiments, the AO-organoid formation phase comprises culturing cells in an AO-organoid medium comprising one or more components as set out in Table 1, optionally at the concentrations shown in Table 1.

In some embodiments, the AO-organoid medium comprises at least R-spondin, a BMP inhibitor, a TGF-beta inhibitor, FGF and heregulin beta-1.

In some embodiments, the step of culturing the lung cells and/or AO-organoid comprises culturing the cells in contact with an exogenous extracellular matrix (such as a basement membrane extract or MatrigelTM).

In some embodiments, the AO-organoid is a 3D organoid.

In some embodiments, the PD-organoid is a 3D organoid.

In some embodiments, the PD-organoid is a 2D organoid.

In some embodiments, wherein PD-organoid is a 2D organoid, the step of culturing in a proximal differentiation medium comprises culturing in a transwell culture system comprising an apical and basal chamber.

In some embodiments, the PD-organoid is a 3D PD-organoid and the method comprises:
a. culturing lung cells from a subject in an AO-organoid formation phase in an AO-organoid medium in contact with an extracellular matrix for a period of time sufficient to generate a 3D AO-organoid, for example for at least 2 days; and
b. changing the AO medium to a proximal differentiation medium supplemented with a notch inhibitor and culturing the 3D AO-organoid in the proximal differentiation medium supplemented with a notch inhibitor for a period of time sufficient to generate a PD-organoid, for example for at least 5 days, at least 10 days, at least 14 days or at least 16 days.

In some embodiments, the PD-organoid is a 2D PD-organoid and the method comprises the step of:
a. culturing lung cells from a subject in an AO-organoid formation phase in an AO-organoid medium in contact with an extracellular matrix for a period of time sufficient to generate a 3D AO-organoid, for example for at least 2 days;
b. dissociating the 3D AO-organoids into single cell suspension;
c. seeding the dissociated cells in the apical chamber of a transwell culture system;
d. optionally culturing the seeded cells in AO medium for at least 1 day, for example, until the cells reach at least 90% confluence; and
e. culturing the seeded cells in proximal differentiation medium supplemented with a notch inhibitor for a period of time sufficient to generate a 2D PD-organoid, for example for at least 5 days, at least 10 days, at least 14 days or at least 16 days.

In some embodiments, the culture medium is added to both the apical and basal chambers of the transwell culture system.

In some embodiments, the culture medium is refreshed every other day.

In some embodiments, the organoid or cells are human organoids or human cells.

In a second aspect of the invention there is provided, a PD-organoid obtained by a method of the first aspect.

In some embodiments, the PD-organoid has at least 2-fold or at least 3-fold increase in the proportion of ciliated cells when compared to the AO-organoid from which it is derived.

In some embodiments, the PD-organoid is further characterised by serine protease expression, for example, expression of one or more or all of TMPRSS2, TMPRSS4, TMPRSS11D (HAT) and Matriptase

In some embodiments, expression of HAT is at least 1 log10 fold increased relative to its expression in AO-organoids.

In some embodiments, the PD-organoid further comprises one or more or all of the following cell types:
a. basal cells, characterised by P63 and CK5 expression;
b. goblet cells, characterised by MUC5AC expression; and
c. club cells characterised by lack of CC10 and SCGB3A2 expression.

In some embodiments, gene expression is assessed using quantitative PCR of mRNA transcripts normalised with GAPDH

In some embodiments, the PD- organoid further comprises an influenza virus.

In some embodiments, the PD-organoid has any one or more of the properties described in any one of claims 9 to 10.

In a third aspect of the invention, there is provided, a method for contracting an influenza virus in a PD-organoid, wherein the method comprises:
a. generating a PD-organoid in accordance with the first or second aspects; and
b. infecting the PD-organoid with an influenza virus.

In some embodiments, the infecting step comprises inoculating with the influenza virus at a multiplicity of infection of at least 0.001, at least 0.01or between 0.001 and 0.01.

In some embodiments, the infecting step further comprises incubating for at least 30 minutes, at least 60 minutes, at least 90 minutes or at least 120 minutes.

In some embodiments, the incubating step is performed at about 37°C.

In some embodiments, the contacting step is at the apical surface of the PD-organoid.

In some embodiments, the PD-organoid is a 2D organoid and the contacting step involves adding the influenza virus to the apical chamber of the transwell culture system.

In some embodiments, the PD-organoid is a 3D organoid and the method further comprises a step of exposing the apical surface of the 3D organoid, for example by mechanical shearing, prior to contacting the PD-organoid with an influenza virus.

In some embodiments, the method further comprises re-contacting the 3D-organoid with an extracellular matrix and culturing the PD-organoid in a proximal differentiation medium, after infecting,

In some embodiments, the method further comprises incubating, the PO-organoid with the influenza virus.

In some embodiments, the method comprises a method for predicting infectivity of the influenza virus to humans and the influenza virus is a test influenza virus, wherein the PD-organoid is a human organoid and wherein the method further comprises:
b. contacting the human PD-organoid with the test influenza virus as claimed;
c. testing the viral titre after a time period sufficient to allow viral propagation.

In some embodiments, the method comprises comparing the viral titre to a control influenza virus.

In some embodiments, testing the viral titre involves detecting a change in viral titre.

In some embodiments, an increase in viral titre is indicative of likely infectivity of the influenza virus to humans and/or wherein a greater increase over a shorter time period is correlated with a higher degree of infectivity.

In some embodiments, the increase in viral titre is at least 1log10 units, at least 2log10 units, or at least 3log10 units within 24 hours.

In some embodiments, the control influenza virus is a known poorly-infective-to-humans influenza virus.

In some embodiments, the change in viral titre of the test influenza virus is greater than the change in viral titre of the known poorly-infective-to-humans influenza virus, for example, wherein the viral titre is at least 10-fold, at least 50-fold, at least 100 fold, at least 1,000 fold or at least 10,000 fold greater than the viral titre of the known poorly-infective-to-humans influenza virus.

In some embodiments, the known poorly-infective influenza virus is selected from H7N2, H9N2 and H9N9.

In some embodiments, the control influenza virus is a known infective-to-humans influenza virus.

In some embodiments, the change viral titre of the test influenza virus is about the same or greater than the viral titre of the known infective-to-humans influenza virus, for example, at least 75%, at least 80%, at least 90%, at least 100%, at least 150%, at least 2-fold, at least 5-fold or at least 10-fold relative to the viral titre of the known infective-to-humans influenza virus.

In some embodiments, the known infective influenza virus is H7N9.

In some embodiments of the second or third aspect, the influenza virus is:
a. an influenza A virus;
b. a human, avian or swine influenza virus; and/or
c. an emerging influenza

### BRIEF DESCRIPTION OF THE DRAWINGS

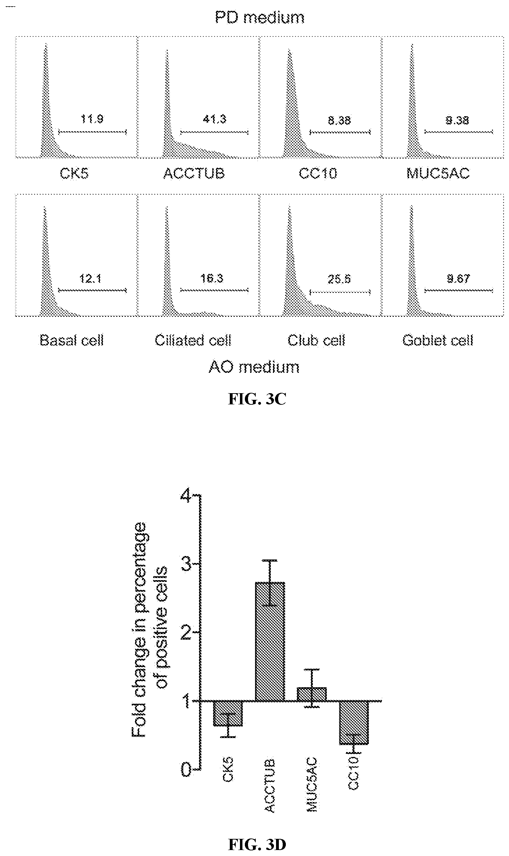

Any of the following figures that do not fall within the scope of the claims are provided for comparative or illustrative purposes only.

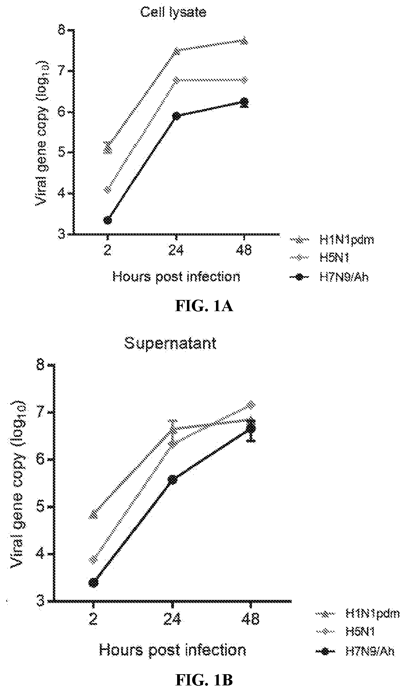

**Figs. 1A-1C** show viral loads in airway organoids inoculated withHlNlpdm, H5N1 and H7N9/Ah. The airway organoids were inoculated with HlNlpdm, H5N1 and H7N9/Ah at an MOI of 0.01. The infected organoids (cell lysate) and supernatants were harvested at the indicated hours to detect the viral loads. Supernatant samples were used for viral titration. Data showing mean ± SD of triplicated samples.

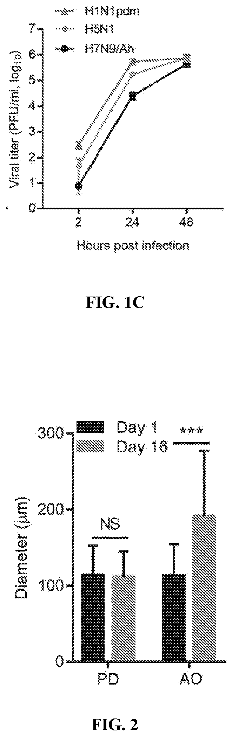

**Fig. 2** shows the diameters of individual organoids. The images of organoids cultured in PD medium and AO medium are used to measure the diameters of individual organoids (n=300) using Image J. Student's T test was used for data analysis. ***, P<0.005.

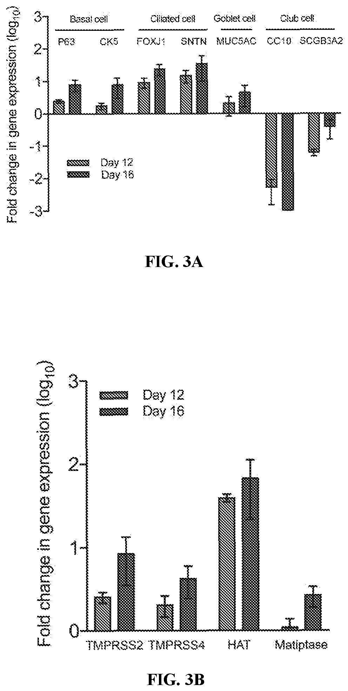

**Figs. 3A-D**show characterization of the differentiation status of airway organoids. (Fig. 3A and 3B). Fold changes in expression levels of cell type markers (Fig. 3A) and serine proteases (Fig. 3B) in the organoids cultured in PD medium versus those in AO medium at the indicated day. Data show mean and
SD of two lines of organoids. Fig. 3C shows the percentages of individual cell types in the organoids cultured in PD medium and AO medium. The representative histograms of one organoid line are shown. Fig. 3D shows fold changes in positive cell percentages in the organoids cultured in PD medium versus those in AO medium.

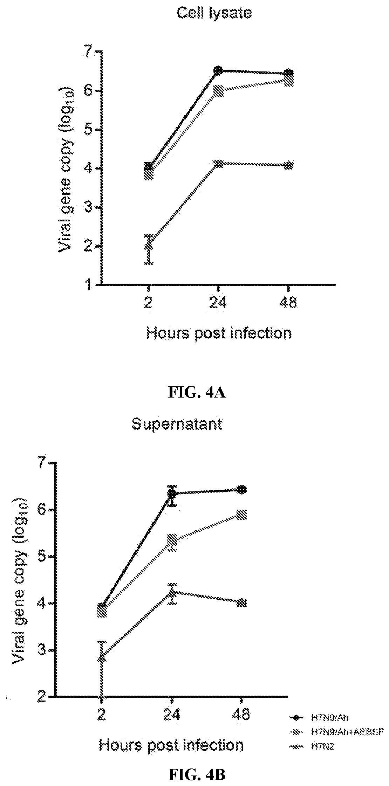

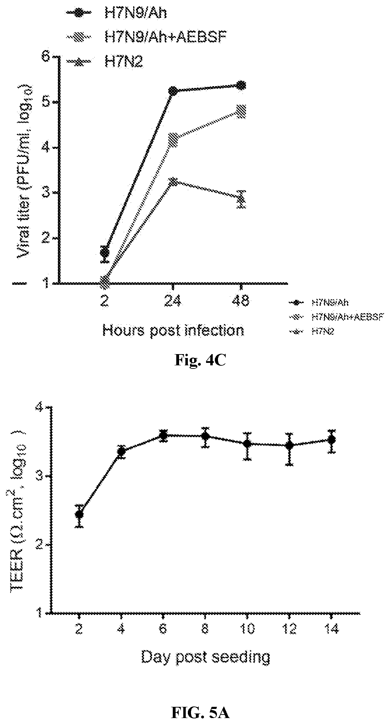

Figs. 4A-C. Influenza virus infection in the 3D PD airway organoids. The 3D PD airway organoids were inoculated with H7N9/Ah and H7N2 virus at an MOI of 0.01. The infected organoids (cell lysate) and supernatants were harvested at the indicated hours to detect the viral loads (Fig. 4A and 4B). Supernatant samples were used for viral titration (Fig. 4C). Data showing mean ± SD of triplicated samples in one representative experiment repeated 3 times.

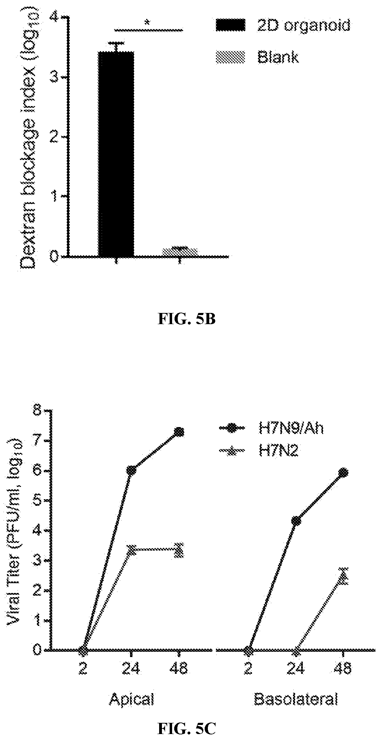

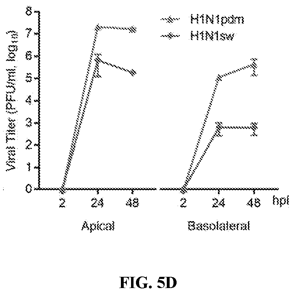

**Figs. 5A-B**show formation of epithelial barrier in 2D monolayers of differentiated airway organoids in transwell culture. The 3D airway organoids were dissociated into single cells, seeded in transwell inserts and cultured in AO medium. At day 2, AO medium was replaced with PD medium. (A) Trans-epithelial electronic resistance (TEER) was measured at the indicated day post seeding. Data show the cell-specific TEER (mean ± SD) of 2D monolayers in 10 inserts. (B) At day 10 after transwell culture, Fluorescein isothiocyanate-dextran (MWlOk) was added in the medium of upper chamber and incubated for 4 hours. The medium in the upper and bottom chamber were collected and applied to fluorescence assay. Dextran blockage index refers to the fluorescence intensity of the medium in the upper chamber versus that in the bottom chamber. Data represent mean and SD of 10 inserts seeded with 2D airway organoids and those in two blank inserts. **Figs. 5C** and **5D** show replication capacity of influenza viruses in established 2D differentiated airway organoids. 2D PD airway organoids were inoculated in duplicate with H7N9/Ah, H7N2 as well as HlNlpdm, HlNlsw at an MOI of 0.001. The cell-free media were harvested from apical and basolateral chambers at the indicated hours post infection (hpi) for viral titration.

### DETAILED DESCRIPTION OF THE INVENTION

### I. Definitions

A "base media," as used herein, refers to a basal salt nutrient or an aqueous solution of salts and other elements that provide cells with water and certain bulk inorganic ions essential for normal cell metabolism and maintains intra-cellular and/or extra-cellular osmotic balance.

An ErbB3/4 ligand is herein defined as a ligand that is capable of binding to ErbB3 and/or ErbB4.

The term "Induced pluripotent stem cell" (iPSC), as used herein, is a type of pluripotent stem cell artificially derived from a non-pluripotent cell.

"Media" or "culture media" as used herein refers to an aqueous based solution that is provided for the growth, viability, or storage of cells used in carrying out the present invention. A media or culture media may be natural or artificial. A media or culture media may include a base media and may be supplemented with nutrients (e.g., salts, amino acids, vitamins, trace elements, antioxidants) to promote the desired cellular activity, such as cell viability, growth, proliferation, and/or differentiation of the cells cultured in the media.

"Organoid" as used herein refers to an artificial, in vitro construct derived from adult stem cells created to mimic or resemble the functionality and/or histological structure of an organ or portion thereof.

The term "pluripotency" (or pluripotent), as used herein refers to a stem cell that has the potential to differentiate into any of the three germ layers: endoderm (for example, interior stomach lining, gastrointestinal tract, the lungs), mesoderm (for example, muscle, bone, blood, urogenital), or ectoderm (for example, epidermal tissues and nervous system).

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other embodiments the values may range in value either above or below the stated value in a range of approx. +/- 5%; in other embodiments the values may range in value either above or below the stated value in a range of approx. +/- 2%; in other embodiments the values may range in value either above or below the stated value in a range of approx. +/- 1%. The preceding ranges are intended to be made clear by context, and no further limitation is implied. All methods described herein can be performed in any suitable order unless otherwise indicated herein or otherwise clearly contradicted by context.

No language in the specification should be construed as indicating any non-claimed element as essential to the practice of the invention.

### II. Compositions

2D and 3D airway organoids are provided, differentiated by culture of cells obtained from lung tissue, in AO culture medium followed by culture in PD cell culture medium. An organoid is a cellular cluster derived from stem cells or primary tissues and exhibits endogenous organ architecture. *See,* e.g., Cantrell and Kuo, Genome Medicine 7:32-34 (2015). Organoids differ from naturally occurring in vivo tissues and from ex vivo tissue explants because they are derived from expansion of epithelial tissue cells only.

The disclosed 3D and 2D differentiated airway organoids support active replication of human infective H7N9/Ah and HlNlpdm. In contrast, the H7N2 virus, which has been temporally and spatially co-circulating with H7N9 viruses in domestic poultry and contains the similar internal genes as H7N9 viruses, replicated much less efficiently in both models. Similarly, the swine H1N1 isolate showed a lower growth capacity than its counterpart of human-adapted HlNlpdm (Fig.5B). Thus, these PD airway organoids discriminate human infective viruses from poorly infective viruses.

In particularly preferred embodiments cells the disclosed 2D and 3D organoids do not recombinantly express of Oct3/4, Sox2, Klf4, c-Myc, L-MYC, LIN28, shRNA for TP53 or combinations thereof, i.e., the 2D and 3D organoids do not include cells genetically engineered to Oct3/4, Sox2, Klf4, c-Myc, L-MYC, LIN28, shRNA for TP53 or combinations thereof.

### A. 3D PD-airway organoids

*In vitro* 3D airway organoids are disclosed. The airway organoids are 3D cysts lined by polarized epithelium. The disclosed airway organoids include a combination of basal cells, ciliated cells, goblet cells, and club cells, and accordingly, express one or more markers selected from the group consisting of ciliated cell markers (FOXJ1 and SNTN), basal cell markers (P63, CK5); goblet cell marker (MUC5AC) and serine proteases including TMPRSS2, TMPRSS4, TMPRSS11D (HAT) and Matriptase. Ciliary beating plays essential roles in human airway biology and pathology, and 50%-80% of airway epithelial cells are ciliated (Yaghi, et al, Cells, 5(4): pii:E402016)). The data in this application demonstrates that the ability to obtain airway organoids with a ciliated cell population that approaches physiological levels (i.e., more than 40% of the total population of organoid cells), depends on the cell culture medium selection (i.e. the factors used to supplement basal medium) as well as the cell culture protocol used to culture cells obtained from lung tissue i.e., timing of when cells are exposed cells to the combination of factors used to supplement basal medium). In a particularly preferred embodiment, the 3D PD-airway organoids contain no type I and type II alveolar epithelial cells in contrast to whole lung tissue, and the cilia on the PD-organoids beat synchronously. The disclosed organoids, generated from in *vitro culture* using a combination of AO and PD culture medium (PD-organoids) show improved expression of these markers, when compared to airway organoids generated from in *vitro culture* in AO culture medium alone (AO-organoids) for the same length of time. Criteria indicating an improvement in morphology and differentiation include for example, an increase in the percentage of ciliated cells following culture in PD medium. When compared to 3D AO-organoids, PD-organoids contain an increased level of ciliated and goblet cells, for example, a 2 fold, to 100 fold increase. In one preferred embodiment, PD organoids are disclosed which include ciliated cells with a near-physiological abundance at a percentage greater than 25% . For example, ciliated cells can make up at least 40% of the cells in the organoid, at day 16 post PD cell culture. Thus, the PD organoids contain about 40% ciliated cells, preferably, between 40 and 50% ciliated cells at day 16 post PD medium cell culture. Meanwhile 3D PD-organoids contain a decreased level of club cells when compared to 3D AO-organoids.

PD-organoids show reduced expression of Club cell markers (CC10, SCGB3A2) compared to AO-organoids.

### B. 2D Differentiated airway organoids

2D PD airway monolayers are provided, with an intact epithelial barrier to allow exclusive apical exposure. The presence of an intact epithelial barrier is determined for example using Transepithelial electrical resistance (TEER). Stabilization of TEER measurement shows formation of an intact barrier as shown for Example in Fig. 5A (shows stabilization of TEER at day 6). The electrical resistance of a cellular monolayer, measured in ohms, is a quantitative measure of the barrier integrity. Other methods of measuring monolayer integrity are known in the art. Reviewed in Elbrecht, et al, J. Rare Disease and Treat. 1(3):46-52 (2016); benson, et al., Fluids Barriers CAN, 10:5 (2013).

A limitation of 3D organoids for studying microbial infections is the inaccessibility of apical surface to pathogens since most organoids are orientated inwards, while receptors for most respiratory viruses are distributed in the apical surface. For virus inoculation, organoids have to be sheared to enable sufficient apical exposure to the virus inoculum (Drumond, et al, P.N.A.S., 114(7): 1672-2677 (2017)).

The disclosed 2D PD organoids include an apical side and a basolateral side. Cells in the 2D organoid include a combination of basal cells, ciliated cells, goblet cells, and club cells, and accordingly, express one or more markers selected from the group consisting of ciliated cell markers (FOXJ1 and SNTN), basal cell markers (P63, CK5); goblet cell marker (MUC5AC) and serine proteases including TMPRSS2, TMPRSS4, TMPRSS11D (HAT) and Matriptase. In a particularly preferred embodiment, the 3D PD-airway organoids contain no type I and type II alveolar epithelial cells.

### III. Methods of making Airway Organoids

The disclosed methods outline steps for culturing cells obtained from lung tissue to generate 3D organoids.

Airway adult stem cell (ASC)-derived organoids disclosed herein, once established, can be expanded indefinitely while displaying remarkable phenotypic and genotype stability. They thus overcome the reproducibility and availability limitations of the current *in vitro* model systems. Several lines of airway organoids were obtained from small pieces of normal lung tissue adjacent to the diseased tissue from patients undergoing surgical resection for clinical conditions. These airway organoids, 3D cysts lined by polarized epithelium, include the four major types of airway epithelial cells, i.e. ciliated cell (ACCTUB+ or FOXJ1+), basal cell (P63+), goblet cell (MUC5AC+), and Club cell (CC10+) (Fig. 1A). The cell culture media used to generate the airway organoids in some preferred embodiments does not include BMP (bone morphogenic protein) 4. In one preferred embodiment, generating a line of 3D organoids from primary lung tissues in AO culture medium disclosed herein takes preferably between one and four weeks, more preferably, between 2 and 3 weeks.

### A. 3D PD-airway organoids

One embodiment provides a method of making an organoid from a mammalian tissue in vitro comprising: (a) obtaining a lung tissue sample from a subject, (b) isolating cells from the mammalian tissue to provide isolated cells by subjecting the tissue sample into single cells; (c) culturing the cells in an airway organoid (AO) culture medium for at least one to four weeks, preferably between 2 and 3 weeks to generate 3D airway organoids. The established 3D airway organoids can be maintained in AO medium and passaged every two to three weeks. (d) and preparing (adjusting) the established 3D airway organoids to an appropriate state (e) culturing the 3D airway organoids in a proximal differentiation medium (PD), for a time sufficient to produce differentiated airway organoids comprising a cell population consisting of at least 25%, at least 30%, at least 35% or at least 40% ciliated cells, wherein the ciliated cells are characterised by FOXJ1 and SNTN expression and wherein the proximal differentiation medium is supplemented with DAPT at a concentration of between 10 and 20 µM. In step (d), the 3D airway organoids are split and maintained in AO medium for at least 2 to 16 days, for example 2 days, 3 days, 4 days, 5 days, 6 days, 7 days, 8 days, 9 days, 10 days, 11 days, 12 days, 13 days, 14 days, 15 days or 16 days. Steps (c) and (d) are preferably three dimensional (3D) cell culture, as opposed to 2D cell culture. While the 2D culture usually grows cells into a monolayer on glass or, more commonly, tissue culture polystyrene plastic flasks, 3D cell cultures grow cells into 3D aggregates/spheroids using a scaffold/matrix. Commonly used scaffold/matrix materials include biologically derived scaffold systems and synthetic-based materials.

In some preferred embodiment, the method is performed with a commercially using extracellular matrix. In some preferred embodiment, the method is performed with a commercially available extracellular matrix such as MATRIGEL^{™} (growth Factor Reduced Basement Membrane Matrix). Other extracellular matrices (ECM) are known in the art for culturing cells. A preferred ECM for use in a method of the invention includes at least two distinct glycoproteins, such as two different types of collagen or a collagen and laminin. In some preferred embodiment, the method is performed with a commercially available extracellular matrix such as MATRIGEL^{™} (growth Factor Reduced Basement Membrane Matrix), which comprises laminin, entactin, and collagen IV. In general, an extracellular matrix comprises laminin, entactin, and collagen. In a preferred embodiment, the method is performed using a 3-dimensional culture device (chamber) that mimics an in vivo environment for the culturing of the cells, where preferably the extracellular matrix is formed inside a plate that is capable of inducing the proliferation of stem cells under hypoxic conditions. Such 3-dimensional devices are known in the art. Other commercially available products include Cultrex^{®} basement membrane extract (BME; Trevigen), and hyaluronic acid are commonly used biologically derived matrixes. Polyethylene glycol (PEG), polyvinyl alcohol (PVA), polylactide-co-glycolide (PLG), and polycaprolactone (PLA) are common materials used to form synthetic scaffolds. Scaffold-free 3D cell spheroids can be generated in suspensions by the forced floating method, the hanging drop method, or agitation-based approaches. Edmondson, et al, Assay Drug. Dev. Technol, 12(4):207-218 (2014). For example, the isolated cells are embedding in 60% MATRIGEL^{™} and seeded in a suspension culture plate prior to culture in the (AO) medium.

In still another preferred embodiment, the AO culture medium step does not include cells expressing Oct4 and/or are not genetically engineered to express one or more markers of pluripotency i.e., the cells iPSC, for example, adult cells induced to pluripotency by expression of Oct3/4, Sox2, Klf4, c-Myc, L-MYC, LIN28, shRNA for TP53 or combinations thereof, or embryonic stem cells, for example, H9 hESCs (Thomson et al., Science 282:1145-1 147 (1998)), 201B7 (Takahashi et al, Cell, 131(5): 861-72 (2007)), 585A1 or 604A1 hiPSCs (Okita et al, Stem Cells, 31(3):458-66 (2013)).

### (i) Sources for Airway Organoids

The disclosed organoids can be cultured from a tissue sample preferably a lung tissue sample obtained from a mammal, such as any mammal (e.g., bovine, ovine, porcine, canine, feline, equine, primate), preferably a human.

In a preferred embodiment, the lung tissue is not obtained from an embryonic human lung, and is obtained from adult lung.

In one embodiment, single cells are obtained from a tissue sample using a combination of steps that result in single cells. The tissue sample size can range in size from 0.1 cm to 10 cm, for example, between 0.5 and 5 cm, in some preferred embodiments between 0.5 and 1.0 cm in size. Cells may be isolated by disaggregating an appropriate organ or tissue that is to serve as the cell source using techniques known to those skilled in the art. For example, the tissue or organ can be disaggregated mechanically and treated with digestive enzymes and/or chelating agents to release the cells, to form a suspension of individual cells. Enzymatic dissociation can be accomplished by mincing the tissue and treating the minced tissue with one or more enzymes such as trypsin, chymotrypsin, collagenase, elastase, and/or hyaluronidase, DNase, pronase, dispase etc.

In a preferred embodiment, single cells are obtained from the lung tissue sample by mincing a lung tissue sample obtained from a subject, digesting with collagenase for 1 to two hours at 37 oC, followed by shearing using glass Pasteur pipette and straining over a filter, for example, a 100pm cell strainer.

In another preferred embodiment adult stem cells are obtained from lung tissue sample by selecting for cells expressing the Lgr5 and/or receptor, which belong to the large G protein-coupled receptor (GPCR) superfamily. One embodiment includes preparing a cell suspension from lung tissue, contacting the cell suspension with cells expressing the Lgr5 and/or receptor, isolating the Lgr5 and/or 6 binding compound, and isolating the stem cells from the binding compound. Examples of Lgr5 and/or Lgr6 binding compounds include antibodies, such as monoclonal antibodies, that specifically recognize and bind to the extracellular domain of either Lgr5 or Lgr6. ETsing such an antibody, Lgr5 and/or Lgr6-expressing stem cells can be isolated using methods known in the art, for example, with the aid of magnetic beads or through fluorescence-activated cell sorting.

In one preferred embodiment the disclosed method does not include the step of selecting for cells expressing any markers, for example, the Lgr5 and/or receptor, using Lgr5 and/or Lgr6 binding compounds or biomarkers for lung disease, such as CPM (carboxypeptidase M) (Dragavic, et al, Am. J. Respir. Crit Care Med., 152:760-764 (1995). This embodiment contemplates a method of generating airway organoids, that does not include enriching the population of starting cells based on surface marker expression

Isolated cells are further cultured as discussed herein. A preferred cell culture medium is a defined synthetic medium, buffered at a pH of 7.4 (preferably between 7.2 and 7.6 or at least 7.2 and not higher than 7.6) with a carbonate-based buffer, while the cells are cultured in an atmosphere comprising between 5% and 10% C0₂, or at least 5% and not more than 10% C0₂, preferably 5% C0₂.

### (ii) AO culture medium

The cells are cultured in supplemented basal cell culture media. In some embodiments, a base media may include at least one carbohydrate as an energy source and/or a buffering system to maintain the medium within the physiological pH range. Examples of commercially available base media may include, but are not limited to, phosphate buffered saline (PBS), Dulbecco's Modified Eagle's Medium (DMEM), Minimal Essential Medium (MEM), Basal Medium Eagle (BME), Roswell Park Memorial Institute Medium (RPMI) 1640, MCDB 131, Click's medium, McCoy's 5 A Medium, Medium 199, William's Medium E, insect media such as Grace's medium, Ham's Nutrient mixture F-10 (Ham's F-10), Ham's F-12, a-Minimal Essential Medium (aMEM), Glasgow's Minimal Essential Medium (G-MEM) and Iscove's Modified Dulbecco's Medium. A preferred basal cell culture medium is selected from DMEM/F12 and RPMI 1640. In a further preferred embodiment, Advanced DMEM/F12 or Advanced RPMI is used, which is optimized for serum free culture and already includes insulin. In this case, the Advanced DMEM/F 12 or Advanced RPMI medium is preferably supplemented with glutamine and Penicillin/streptomycin. In preferred embodiments, the basal medium comprises Gastrin. In some embodiments, the basal medium also comprises NAc and/or B27.

In some embodiments an AO medium as described in WO2016/083613 can be used. In a particularly preferred embodiment, an AO culture medium (Table 1) is used, which is supplemented base media suitable to maintain airway organoids in culture.

The AO culture medium is base medium supplemented with agents such as Rspondin (a Wnt agonist), a BMP inhibitor, a TGF-beta inhibitor, a fibroblast growth factor (FGF) and Nicotinamide.

In some embodiments, the supplemented basal culture medium used to culture cells dissociated from a tissue sample does not include a GSK3 inhibitor, for example CFHR99021 (6-[[2-[[4-(2,4-Dichlorophenyl)-5-(5-methyl-liT-imidazol-2-yl)-2-pyrimidinyl]amino]ethyl]amino]-3-pyridinecarbonitrile). Known GSK-inhibitors comprise small-interfering RNAs, 6-Bromoindirubin-30-acetoxime.

A preferred AO medium is shown in Table 1.

**Table 1. Composition of human airway organoid (AO) medium.**

| Reagents | Company | Catalog No. | Working concentration |
|---|---|---|---|
| Advanced DMEM/F12 | Invitrogen | 12634010 | n/a |
| HEPES | Invitrogen | 15630-056 | 1% |
| GlutaMAX | Invitrogen | 35050061 | 1% |
| Penicillin-Streptomycin | Invitrogen | 15140-122 | 1% |
| Rspondin1* (conditioned medium) | n/a | n/a | 10% |
| Noggin* (conditioned medium) | n/a | n/a | 10% |
| B27 supplement | Invitrogen | 17504-044 | 2% |
| N-acetylcysteine | Sigma | A9165 | 1.25mM |
| Nicotinamide | Sigma | N0636 | 10mM |
| Y-27632 | Tocris | 1254 | 5µM |
| A8301 | Tocris | 2939 | 500nM |
| SB202190 | Sigma | S7067 | 1µM |
| FGF-7 | Peprotech | 100-19 | 5ng/ml |
| FGF-10 | Peprotech | 100-26 | 20ng/ml |
| Primocin | InvivoGen | ant-pm-1 | 100µg/ml |
| Heregulin beta-1 | Peprotech | 100-03 | 5nM |

| | | | |
|---|---|---|---|
| *Conditioned media were produced from stable cell lines for production of R-spondinl and Noggin. | | | |

The AO medium incudes a BMP inhibitor. BMP inhibitor is defined as an agent that binds to a BMP molecule to form a complex wherein the BMP activity is neutralized, for example by preventing or inhibiting the binding of the BMP molecule to a BMP receptor. Alternatively, the inhibitor is an agent that acts as an antagonist or reverse agonist. BMP-binding proteins that can be used in the disclosed methods include, but are not limited to Noggin (Peprotech), Chordin and chordin-like proteins (R&D sytems) comprising chordin domains, Follistatin and follistatin-related proteins (R&D sytems) comprising a follistatin domain, DAN and DAN-like proteins (R&D sytems) comprising a DAN cysteine-knot domain, sclerostin/SOST (R&D sytems), decorin (R&D sytems), and alpha-2 macroglobulin (R&D systems). Most preferred BMP inhibitor is Noggin. Noggin is preferably added to the basal culture medium at a concentration of at least about 10%.

The AO medium incudes a WNT agonist. Wnt agonists include the R-spondin family of secreted proteins, which is include of 4 members (R-spondin 1 (NU206, Nuvelo, San Carlos, Calif.), R-spondin 2 ((R&D sytems), R-spondin 3, and R-spondin-4); and Norrin.. In a preferred embodiment, a Wnt agonist is selected from the group consisting of: R-spondin, Wnt-3a and Wnt-6. Preferred concentrations for the Wnt agonist are about 10% for R-spondin and approximately 100 ng/ml or 100 ng/ml for WNt-3a. In some preferred embodiments, the WNT agonist is not a GSK inhibitor.

SB202190 (4-(4-Fluorophenyl)-2-(4-hydroxyphenyl)-5 -(4-pyridyl)- 1H-imidazole) is a highly selective, potent and cell permeable inhibitor of p38 MAP kinase. SB 202190 binds within the ATP pocket of the active kinase (¾ = 38 nM, as measured in recombinant human p38), and selectively inhibits the p38a and β isoforms. Other useful p38 MAPK inhibitors include, but are not limited to SB203580 (4-[5-(4-Fluorophenyl)-2- [4-(methylsulfonyl)phenyl]-1 _{*H* -}imidazol-4-yl]pyridine); SB 203580 hydrochloride (4-[5-(4-Fluorophenyl)-2-[4-(methylsulphonyl)phenyl]- 1//-imidazol-4-yl]pyridine hydrochloride); SB202190 (4-[4-(4-Fluorophenyl)-5-(4-pyridinyl)- 1//-imidazol-2-yl]phenol); DBM 1285 dihydrochloride (/V-Cyclopropyl-4-[4-(4-fluorophenyl)-2-(4-piperidinyl)-5-thiazolyl]-2-pyrimidinamine dihydrochloride); SB 239063 (/ra«.s-4-[4-(4-Fluorophenyl)-5-(2-methoxy-4-pyrimidinyl)-1 //-imidazol-1 -yl]cyclohexanol); SKF 86002 dihydrochloride (6-(4-Fluorophenyl)-2,3-dihydro-5-(4-pyridinyl)imidazo[2, 1Ajthiazole dihydrochloride).

A8301 (3-(6-Methyl-2-pyridinyl)-iV-phenyl-4-(4-quinoliny!)-li/- pyrazole-1-carbothioamide) is potent inhibitor of TGF-β type I receptor ALK5 kinase, type 1 aetiviminodal receptor ALK4 and type 1 nodal receptor ALK7. A83-01 may be added to the culture medium at a concentration of between 10 nM and 10 uM, or between 20 nM and 5 uM, or between 50 nM and 1 uM. For example, A83-01 may be added to the culture medium at approximately 500 nM. Other useful TGF-β type I receptor inhibitors include, but are not limited to SB431542 (4-[4-(1,3-benzodioxoi-5-yl)-5-(2-pyridinyl)-lfi-imidazol-2-yljbenzamide): LY 364947 (4-[3-(2-Pyridinyi)-li/-pyrazol-4-yl]-quinoline); R 268712 (4-[2-Fluoro-5-[3-(6-methyl-2-pyridinyl)-1/7-pyrazol-4-yl]phenyi]-1//- pyrazole-l -ethanol); SB 525334 (6-[2-(1,1-Dimethylethyl)-5-(6-methyl-2-pyridinyl)-I//-imidazol-4-yj]quinoxaline); and SB 505124 (2~[4~(1,3-Benzodioxol-5-yl)-2-(l ,1-dimethylethyl)- 1/f~imidazo3-5-yl]-6-methyl-pyridine) Y-27632 (/ran.v-4-[(ii?)-l-Aminoethyl]-A -4-pyridinylcye3ohexanecarboxamide dihydrochloride) is a selective pl6QROCK inhibitor. Other useful Rho inhibitors include isoquinolin and (S)-(+)-2-methyl-1-[(4-methyl- 5-isoquinolinyl)sulfonyl] -hexahydro- 1H-1,4- diazepine dihydrochloride (H-l 152; Tocris Bioscience).

In particularly preferred embodiments, the AO or PD cell culture media used in the disclosed methods includes an ErbB3/4 ligand (e.g. human neuregulin β-1). The ErhB receptor tyrosine kinase family consists of four cell surface receptors, ErbBl/EGFR HER1 , li) ErhB2/HER2, iii) ErbB3/HER3, and iv) ErbB4/HER4. ErbB3/4 ligands include members of the neuregulin/heregulm family. The neuregulin/heregulin family is referred to herein as the neuregulin family. The neuregulin family is a family of structurally related polypeptide growth factors that are gene products of alternatively spliced genes NRG1, NRG2, NRG3 and NRG4. In more preferred embodiments, the excluded one or more ErbB3/4 ligands of the culture medium are polypeptides that are gene products of one or more of NRG1, NRG2, NRG3 and NRG4 {i.e. a neuregulin polypeptide).

### (Hi). PD Culture medium

A preferred PD medium is a cell culture medium suitable for air-liquid interface culture of bronchial epithelial cells. In some embodiments, the PD medium comprises one or more (or all) of the components listed in Table 2, preferably at the concentrations shown in Table 2.

**Table 2. Composition of PD medium.**

| **PD medium components** | **Exemplary concentrations** |
|---|---|
| Basal medium | 50:50 mixed LHC basal medium and DMEM medium supplemented with retinoic acid (50nM) |
| EGF | 0.5ng/ml |
| bovine serum albumin | 150mg/ml |
| bovine pituitary extract | 10ug/ml |
| insulin | 5ug/ml |
| transferrin | 10ug/ml |
| hydrocortisone | 72ng/ml |
| triiodothyronine | 6.7ng/ml |
| epinephrine | 0.6ug/ml |
| antibiotics | Penicillin-Streptomycin (100U/ml), Gentamicin (50ug/ml) and/or Amphotericin B (0.25ug/ml) |

In some embodiments, the PD medium is serum free and/or BPE (bovine pituitary extract)-free. An example of a suitable PD medium is the commercially available PneumaCult-ALI medium (StemCell Technologies). PneumaCult^{™}-ALI Medium is a serum- and BPE-free medium for the culture of human airway epithelial cells at the air-liquid interface (ALI). Airway epithelial cells cultured in PneumaCult^{™}-ALI Medium undergo extensive mucociliary differentiation to form a pseudostratified epithelium that exhibits morphological and functional characteristics similar to those of the human airway *in vivo.* PneumaCult^{™}-ALI Medium supports the generation of differentiated airway organoids in a 2D or 3D culture system.

The PD media is supplemented with DAPT between 10 and 20 µM and more preferably about 10 µM.

Examples of Notch inhibitors not claimed gamma-secretase inhibitors, such as dibenzazepine (DBZ) or benzodiazepine (BZ) or LY-411575, an inhibitor capable of diminishing ligand mediated activation of Notch (for example via a dominant negative ligand of Notch or via a dominant negative Notch or via an antibody capable of at least in part blocking the interacting between a Notch ligand and Notch), or an inhibitor of ADAM proteases. notch inhibitor is DAPT ([N-(N-[3,5-difluorophenacetyl]-L-alanyl)-S-phenylglycine t-butyl ester).

The isolated cells cultured in AO medium are subsequently cultured in PD medium for a period of time effective for formation of PD-organoids. In one preferred embodiment, the time period of time effective for formation of PD-organoids is from about five to about 20 days. In another preferred embodiment, the period of time effective for formation of airway organoids is about 14 days.

### B. 2D PD organoids

2D PD organoids may be obtained from 3D airway organoids by a method that includes dissociating the 3D AO into a single cell suspension, seeding the cells in transwell inserts and culturing the cells in AO medium followed by culture in PD medium for a period of time effective for formation of an intact epithelial barrier, as measured for example, by a dextran penetration assay. The 3D organoids are dissociated into single cells using methods known in the art (discussed herein), preferably, by enzymatic dissociation, followed by shearing and straining over a filter as disclosed in the Examples.

The dissociated cells are cultured as a monolayer, preferably on a permeable support (cell culture insert) in AO medium at 37°C in a humidified incubator with 5% C0 ₂ for 1~2 days and then cultured in PD medium as a monolayer for a time period between 5 and 16 days, preferably between 10 and 14 days, and more preferably, for about 12- 14 days to obtain 2D PD-organoids. The PD medium is preferably provided on the apical and basolateral sides of the monolayer. Permeable supports are commercially available, for example, Corning^{®} Transwell^{®}. Transwell inserts are convenient, ready-to-use permeable support devices pre-packaged in standard multiple well plates. The unique, self-centered hanging design prevents medium wicking between the insert and outer well. Transwell inserts are available in a wide variety of sizes, membrane types, and configurations.

### TV. Methods of using the Composition

The disclosed 3D and 2D proximal differentiated airway organoids can morphologically and functionally simulate human airway epithelium.

Organoids derived from adult stem and progenitor cells reliably retain their in vivo regenerative activity in vitro, and thus provide detailed snapshots of tissue restoration after injury. Lung organoids allow researchers to study processes governing homeostatic regulation of lung tissue and screen factors that impact lineage-specification of stem cells.

Accordingly, the disclosed PD-organoids may be used as an alternative to live animal testing for compound or for treatment of (including resistance to treatment of) lung infection or disease (e.g., chronic obstructive pulmonary disease (COPD)).

Influenza virus infection represents a major threat to public health worldwide. The disclosed 3D and 2D proximal differentiated airway organoids can morphologically and functionally simulate human airway epithelium and can discriminate human infective influenza viruses from poorly infective viruses. Thus, the proximal differentiated airway organoids can be utilized to determine the infectivity of influenza viruses and significantly extend advances in influenza research and provide solutions to influenza infection. One of the most important and challenging issues for infectious disease research, for example, influenza research is to predict which animal or emerging influenza virus can infect humans. In one embodiment, a method for determining infectivity of a influenza virus in humans, by comparing infectivity of the non-human virus in the disclosed 3D or 2D differentiated airway organoids, and comparing its infectivity with a strain of that pathogen known to be highly infectious in humans (high infectivity control) and a strain of that pathogen known have no or low infectivity in humans (low-infectivity control). For example, human infective H7N9/Ah and HlNlpdm can be used as positive control and H7N2 or swine H1N1 used as negative control to determine compare their replication in the 2D or 3D organoids compared to the virus whose infectivity in humans is being tested. Replication in the 2D or 3D organoid comparable with H7N9/Ah and HlNlpdm, indicates that the virus being tested would be infective in humans. Conversely, replication comparable to H7N2 or swine H1N1 indicates that the virus being tested would be low infectivity in humans.

For acute treatment testing, compound or vaccine may be applied to the PD-organoid, e.g., once for several hours. For chronic treatment testing, compound or vaccine may be applied, e.g., for days to one week. Such testing may be carried out by providing an airway organoid product as described herein under conditions which maintain constituent cells of that product alive (e.g., in a culture media with oxygenation); applying a compound to be tested (e.g., a drug candidate) to the lung PD-organoid (e.g., by topical or vapor application to the epithelial layer); and then detecting a physiological response (e.g., damage, infection, cell proliferation, cell death, marker release such as histamine release, cytokine release, changes in gene expression, etc.), the presence of such a physiological response indicating said compound or vaccine has therapeutic efficacy, toxicity, or other metabolic or physiological activity if inhaled or otherwise delivered into the airway of a mammalian subject. A control sample of the PD-organoid may be maintained under like conditions, to which a control compound (e.g., physiological saline, compound vehicle or carrier) may be applied, so that a comparative result is achieved, or damage can be determined based on comparison to historic data, or comparison to data obtained by application of dilute levels of the test compound, etc.

In some preferred embodiment, the disclosed PD-organoid is can be used for influenza virus testing (infectivity and vaccines). In a particularly preferred embodiment, the disclosed PD-organoid can discriminate human infective influenza viruses from poorly infective viruses. Thus, the proximal differentiated airway organoids can be utilized to predict the infectivity of influenza viruses and significantly extend the current armamentaria of influenza research toolbox.

Pre-clinical models of human disease are essential for the basic understanding of disease pathology and its translational application into efficient treatment for patients. Patient-derived organoid cultures from biopsies and/or surgical resections can be used for personalized medicine. Two examples are lung cancer and cystic fibrosis. Additionally, tissue samples can be obtained from a subject cultured as disclosed herein and used to determine the subject's responsiveness to medication in order to select the better treatment for that subject. Dekkers et al. Science Translational Medicine, 8(344):344ra84 (2016*)* showed that the efficacy of cystic fibrosis transmembrane conductance regulator (CFTR)-modulating drugs can be individually assessed in a laboratory test using epithelial cells cultured as mini-guts from rectal biopsies from subjects with cystic fibrosis. The authors show that the drug responses observed in mini-guts or rectal organoids can be used to predict which patients may be potential responders to the drug. Similar preclinical tests using the disclosed 3D organoids obtained from a subject may help to quickly identify responders to CFTR-modulating drug therapy even when patients carry very rare CFTR mutations.

*Ex vivo* expanded adult stem cell-derived organoids retain their organ identity and genome stability, and can be differentiated to PD lung organoids as described herein.

Airway organoids can easily be established from bronchiolar lavage material of humans, allowing inter-individual comparisons; airway organoids can also be readily modified by lentiviruses and CRISPR technologies and can be single cell-cloned. In combination with the molecular toolbox of influenza virologist, the human differentiated airway organoid model system offers great opportunities for studying virus and host factors that define characteristics of this major animal and human pathogen.

The present invention will be further understood by reference to the following non-limiting examples.
Any of the following examples that do not fall within the scope of the claims are provided for comparative or illustrative purposes only.

### V. Examples

### A. Materials and Methods

*Establishing adult stem cell-derived human airway organoids.*

Generation of adult stem cells (ASC) derived human airway organoids was based on the following protocol. Briefly, upon ethical approval by Institutional Review Board of the University of Hong Kong/Hospital Authority Hong Kong West Cluster (HKU/HA HKW IRB, UW 13-364) and informed consents of patients, small pieces of normal lung tissues around 0.5-0.8 cm in size and adjacent to the diseased tissues, were obtained from patients who underwent surgical operation. The tissues were minced and digested with 2mg/ml collagenase (Sigma Aldrich) for 1~2 hours at 37°C, followed by shearing using glass Pasteur pipette and straining over a 100pm filter. The resultant single cells were embedded in 60% MATRIGEL^{™} and were seeded in 24-well suspension culture plate. After solidification, MATRIGEL^{™} droplets containing single cells were maintained with airway organoid (AO) culture medium (Table 1) at 37°C in a humidified incubator with 5% C0 ₂. The organoids were passaged every 2~3 weeks. The bright field images of the organoids were acquired using Nikon Eclipse TS100 Inverted Routine Microscope. To generate PD organoids, airway organoids were split and cultured in AO medium for 2-7 days, following which the culture medium was changed to PD medium.

### Proximal differentiation of human airway organoids.

The airway organoids were split and maintained in AO medium for 2-7 days. The culture media in half of the organoids were changed to proximal differentiation (PD) medium, PneumaCult-ALi medium (StemCell Technologies) supplemented with 10µM DAPT, a notch pathway inhibitor (Tocris). The organoids were then cultured AO or PD media for 16 days, to obtain 3D AO-airway organoids and 3D-PD airway organoids, respectively. Bright field images were taken every 3 days. Diameters of individual organoids were measured with Image! The movies of organoids were acquired using Total Internal Reflection Fluorescent (TIRF) Microscope and Nikon Eclipse Ti2 Inverted Microscope System. At the indicated days, the organoids in the different media were harvested for detection of cellular gene expression or applied to flow cytometry analysis.

### Establishing 2D differentiated airway organoids with transwell culture.

Transwell culture of airway organoids was performed as described elsewhere (24, 25) with modifications. Briefly, the 3D airway organoids were dissociated into single cell suspension after digested with lOxTrypLE^{™} Select Enzyme (Invitrogen) for l~5min at 37°C, sheared using Pasteur pipette and strained over a 40µm filter. Approximately 3.5x1 0⁵ cells were seeded into each transwell insert (Corning, product # 3494). The cells were cultured in AO medium at 37°C in a humidified incubator with 5% C0 ₂ for 1~2 days. When cells reached >90% confluence, the AO medium was changed to PD medium in both the apical and basal chambers. The medium was changed every other day and the cells were maintained for 14 days. Trans-epithelial electronic resistance (TEER) was measured every other day using Millicell ERS-2 Volt-Ohm Meter (EMD Millipore). To assess the integrity of the 2D organoid monolayer as an epithelial barrier, at day 12 after seeding, fluorescein isothiocyanate-dextran with an average molecular weight of 10,000 (Sigma Aldrich) was added in the medium of upper chamber at a concentration of 1 mg/ml and incubated at 37°C for 4 hours. Subsequently, the culture media were harvested from the upper and bottom chambers to detect the fluorescence intensity using the Victor XIII Multilabel Reader (PerkinElmer).

### Propagation of influenza A viruses.

Influenza A virus A/Anhui/l/2013(H7N9) (H7N9/Ah), A/Hong Kong/4 15742/2009(H1N1) (HlNlpdm) and swine H1N1 isolate (HlNsw) were propagated in Madin-Darby Canine Kidney (MDCK) cells. At 72 hours post inoculation (hpi), cell-free medium was harvested, aliquoted and stored at -80°C. Avian IAVs H7N2 and Viet Nam/l 194/04 (H5N1) was propagated in special pathogen-free embryonated chicken eggs at 37°C for 36 hours. The eggs were chilled for overnight at 4°C; then the virus-containing allantoic fluid was harvested, aliquoted and stored at -80°C. Virus titer was determined by plaque assay.

### Influenza A virus infection in human airway organoids.

The 3D airway organoids were sheared mechanically to expose the apical surface to the virus inoculum. The sheared organoids were then incubated with viruses at a multiplicity of infection (MOI) of O.Olfor 2 hours at 37°C. After washing, the inoculated organoids were re-embedded into MATRIGEL^{™} and then cultured in the PD medium. In the H7N9/Ah and H7N2 infection in the 3D PD organoids, one set of H7N9/Ah-inoculated organoids were treated with a serine proteases inhibitor AEBSF (0.125mM, Merck Millipore) during inoculation and after inoculation. At the indicated hpi, organoids, dissolved MATRIGEL^{™} and culture medium were harvested for detection of viral load. The cell-free MATRIGEL^{™} and the culture medium from each droplet were pooled together as one sample, referred as supernatant. The supernatant samples were also used for viral titration. The 2D PD airway organoids were inoculated with the indicated viruses at an MOI of 0.001, from the apical side by adding the virus inoculum into the apical chamber and incubating for 2 hours at 37°C. At the indicated hpi, cell-free media were collected from apical and basolateral chambers for subsequent viral titration. The membranes seeded with 2D organoids were incised from transwell inserts, fixed and applied to immunofluorescence staining as described previously (13).

### RNA extraction, reverse transcription and quantitative polymerase chain reaction (RT-qPCR).

To evaluate the differentiation status of airway organoids cultured in PD medium versus those in AO medium, the organoids were harvested at the indicated hours and applied to RNA extraction using MiniBEST Universal RNA extraction kit (TaKaRa). To evaluate virus replication, the organoids and supernatant samples were lysed for RNA extraction using MiniBEST Universal RNA extraction kit and MiniBEST Viral RNA/DNA Extraction Kit (TaKaRa) respectively. Complementary DNA (cDNA) was synthesized with Transcriptor First Strand cDNA Synthesis Kit (Roche) with Oligo-dT primer. qPCR was performed with LightCycler 480 SYBR Green I Master (Roche) using gene specific primers (Table 3) to detect cellular gene expression level and viral gene copy number. The mRNA expression levels of cellular genes were normalized with that of GAPDH. Viral gene copy number was determined by absolute quantification using a plasmid expressing a conserved region of IAV M gene.

**Table 3. Primers for quantitative PCR assay.**

| Gene Name | Primer Sequence | |
|---|---|---|
| p63 (TP63) | F | CAGACTCAATTTAGTGAGCC (SEQ ID NO:1) |
| | R | CTGCTGGTCCATGCTGTT (SEQ ID NO:2) |
| keratin 5 (KRT5) | F | GAGGAATGCAGACTCAGTGGA (SEQ ID NO:3) |
| | R | TAGCTTCCACTGCTACCTCCG (SEQ ID NO:4) |
| forkhead box J1 (FOXJ1) | F | TCGTATGCCACGCTCATCTG (SEQ ID NO:5) |
| | R | CGGATTGAATTCTGCCAGGT (SEQ ID NO:6) |
| sentan, cilia apical structure protein (SNTN)* | F | GCTGCAAACCCAATTTAGGA (SEQ ID NO:7) |
| | R | TGCTCATCAAGTTCAGAAAGGA (SEQ ID NO:8) |
| mucin 5AC, oligomeric mucus/gel-forming (MUC5AC) | F | CCTACAAAGCTGAGGCCTGT (SEQ ID NO:9) |
| | R | GACCCTCCTCTCAATGGTGC (SEQ ID NO:10) |
| secretoglobin family 1A member 1 (SCGB1A1) | F | AGCATCATTAAGCTCATGGAAAAA (SEQ ID NO:11) |
| | R | GTGGACTCAAAGCATGGCAG (SEQ ID NO:12) |
| secretoglobin family 3A member 2 (SCGB3A2) | F | AACTGCTGGAGGCGCTATCA (SEQ ID NO:13) |
| | R | TGTCCTTTTCACGGGTCACT (SEQ ID NO:14) |
| transmembrane protease, serine 2 (TMPRSS2) | F | CTTTGAACTCAGGGTCACCA (SEQ ID NO:15) |
| | R | TAGTACTGAGCCGGATGCAC (SEQ ID NO:16) |
| transmembrane protease, serine 4 (TMPRSS4) | F | TGCTTCAGGAAACATACCGA (SEQ ID NO:17) |
| | R | CTGGAGTGAGCTCCTCATCA (SEQ ID NO:18) |
| transmembrane protease, serine 11D (TMPRSS11D) | F | TACACAGGAATACAGGACTT (SEQ ID NO:19) |
| | R | CTCACACCACTACCATCT (SEQ ID NO:20) |
| Matriptase | F | CTAGGATGAGCAGCTGTGGA (SEQ ID NO:21) |
| | R | AAGAATTTGAAGCGCACCTT (SEQ ID NO:22) |
| IAV M gene | F | CTTCTAACCGAGGTCGAAACG (SEQ ID NO:23) |
| | R | GGCATTTTGGACAAAKCGTCTA (SEQ ID NO:24) |

### Plaque assay.

Plaque assay was performed to determine titers of the virus stocks and supernatant samples as described elsewhere with minor modification (26). Briefly, MDCK cells were seeded in 12-well plates. Confluent monolayers were inoculated with 200 µL of 10-fold serial dilutions of samples and incubated for 1 hour at 37°C. After removing the inoculum, the monolayers were overlaid with 1% LMP Agarose (Invitrogen) supplemented with MEM and 1pg/pl TPCK-treated Trypsin and further incubated for 2~3 days. The monolayers were fixed with 4% PFA and stained with 1% crystal violet to visualize the plaque after removing the agarose plugs. Virus titers were calculated as plaque-forming units (PFU) per milliliter.

### Immunofluorescence staining

To identify the indicated cell types and the virus-infected cells, the 3D and 2D airway organoids were applied to immunofluorescence staining. Briefly, the organoids fixed with 4% PFA, permeabilized with 0.1-5% Triton X-100 and blocked with Protein block (Dako). Then the organoids were incubated with primary antibodies (Table 4) diluted in Antibody Diluent buffer (Dako) overnight at 4°C, followed by incubation with secondary antibody (Table 4) for 1-2 hours at room temperature. Nuclei and actin filaments were counterstained with 4'-6-diamino-2-phenylindole (DAPI) (Invitrogen) and Phalloidin-647 (Sigma Aldrich) respectively. The confocal images were acquired using Carl Zeiss LSM 780 or 800.

**Table 4. List of Antibodies for used for incubation.**

| Antibodies | Company | Catalog No. |
|---|---|---|
| Mouse Anti-Cytokeratin 5 | Abcam | ab128190 |
| Rabbit Anti-p63 | Abcam | ab124762 |
| Mouse Anti-β-tubulin 4 | Sigma | T7941 |
| Mouse Anti-FOX J1 | Invitrogen | 14-9965-82 |
| Mouse Anti-Mucin 5AC | Abcam | ab3649 |
| Rat Anti-Uteroglobin/CC-10 | R&D Systems | MAB4218-SP |
| Rabbit Anti-Influenza A NP | Novus | NBP2-16965 |
| Goat Anti-Mouse, Alexa Fluor 488 | Invitrogen | A11001 |
| Goat Anti-Mouse Alexa Fluor 594 | Invitrogen | A11005 |
| Goat Anti-Rabbit Alexa Fluor 488 | Invitrogen | A11034 |
| Goat Anti-Rabbit Alexa Fluor 594 | Invitrogen | A11037 |
| Goat Anti-Rat Alexa Fluor 594 | Invitrogen | A11007 |

### Flow cytometry analysis.

To assess the percentage of four types of cells, the airway organoids were applied to flow cytometry analysis. Briefly, the organoids were dissociated with lOmM EDTA (Invitrogen) for 30-60 minutes at 37°C, fixed with 4% PFA and permeabilized with 0.1% Triton- 100. Subsequently, the cells were incubated with primary antibodies (Table 4) for 1 hour at 4°C and followed by secondary antibodies staining. BD FACSCantoII Analyzer was used to analyze the samples.

### Statistical Analysis

Student's t test was used for data analysis. P < 0.05 was considered to be statistically significant.

### B. Results

### Characterization of the human airway organoids.

Several lines of airway organoids (3D cysts lined by polarized epithelium) were established as discussed briefly above, using the OA culture medium, the lung cell culture medium disclosed in U.S. Published Application No. 2017/275592. The four main types of airway epithelial cells were present, i.e. ciliated cell (ACCTUB+ or FOXJ1+), basal cell (P63+), goblet cell (MUC5AC+), and Club cell (CC10+). Apical ACCTUB clearly indicated the orientation of polarization. Most organoids were orientated inwards the lumen; while a small proportion of the organoids were inverted. Beating cilia were visible. No type I and type II alveolar epithelial cells was present. Thus, these organoids resembled the pseudostratified ciliated airway epithelium. The airway organoids were infected by human IAV HlNlpdm, low pathogenic avian virus H7N9/Ah and highly pathogenic avian virus H5N1 (Figs. 1A-1C). The intracellular (cell lysate) viral loads of all 3 virus strains increased over 2 logio units (Fig. 1A). The extracellular (supernatant) viral loads (Fig. 1B), especially the viral titers (Fig. 1C), were elevated by 2~3 logio units.

Ciliary beating plays essential roles in human airway biology and pathology, and 50%-80% of airway epithelial cells are ciliated (Yaghi, et al., Cells, 5(4): pii:E4020l6)). However, by immunostaining and flow cytometry, ciliated cells were apparently under-represented in these airway organoids. Therefore, despite the discernible multi-lineage differentiation and the ability to support replication of IAVs, further improvement of morphology and differentiation appeared required. Furthermore, when these AO-organoids are passaged over time, less and less cilia can be observed. After consecutively passaging 3 months, cilia are not detectable.

### Proximal differentiation of the airway organoids.

To improve proximal differentiation, various protocols and variations thereof were investigated, selecting a proximal differentiation (PD) medium supplemented with DAPT ([N-(N-[3,5-difluorophenacetyl]-L-alanyl)-S-phenylglycine t-butyl ester to induce ciliary differentiation. The organoids in the original airway organoid (AO) medium gradually enlarged, whilst those in PD medium became more compact. After 16 days of culture, the organoids in AO medium grew 2 times larger approximately, while the PD organoids basically remained unchanged (Fig. 2). From day 7, numbers of ciliated cells increased markedly in PD medium. At day 16, beating cilia were observed in a minority of (< 10%) the organoids in AO medium, whilst abundant beating cilia were present in every PD organoid. The synchronously beating cilia drove the cell debris within the organoid lumens to swirl unidirectionally. The dramatically increased abundance of ciliated cells in the PD organoids was verified by immunofluorescence staining. This is in contrast to the 3D organoids obtained from LBO (lung bud organoids) disclosed in Chen, et al, Nat Cell Biol 19(5): 542-549, (2017), where *in vitro* cultures are strongly biased towards distal lung, and, although some areas co-expressing SOX2 and SOX9 expressed more proximal markers for goblet cells and club cell precursors, mature club cells, ciliated cells or basal cells were not observed. Nikolic, et al, Elife 6: e26575 (2017))

Consistently, the transcriptional levels of ciliated cell markers, FOXJ1 and SNTN, were strongly upregulated in the PD organoids compared with the organoids in AO medium. The expression levels of basal cell markers (P63, CK5) and goblet cell marker (MUC5AC) also increased; whereas the levels of Club cell markers (CC10, SCGB3A2) were substantially downregulated in the PD organoids (Figs. 3A and 3B). Importantly, globally elevated expression of serine proteases including TMPRSS2, TMPRSS4, TMPRSS1 1D (HAT) and Matriptase was observed, which are essential for the activation and propagation of human IAVs and low pathogenic avian IAVs (8). Flow cytometry analysis was also performed to measure the percentages of the four cell types in the organoids cultured in two distinct media at day 16. It was shown that, the percentage of ciliated cell remarkably increased around 3-fold after proximal differentiation, to over 40% in the PD organoids; while the ciliated cells invariably constituted a minority of the cells in the organoids in AO medium (Figs. 3C and 3D). Goblet cells also marginally increased; while Club cells consistently decreased after proximal differentiation (Figs. 3C and 3D). Collectively, mucociliary differentiation and developed proximal differentiated airway organoids which can morphologically and functionally simulate human airway epithelium was successfully induced in the original airway organoids.

### Proximal differentiated airway organoids can identify human infective virus.

One of the most important and challenging issues for influenza research is to predict which animal or emerging influenza virus can infect humans. As mentioned above, the novel reassortant avian H7N9 viruses have caused continuing poultry-to-human transmission since 2013. Other subtypes of avian IAVs (including H7N2, H9N2 and H9N9) have been co-circulating with the H7N9 viruses in domestic poultry. These viruses are highly similar in internal genes; yet differ in neuraminidase (NA) or HA and NA (18). Very few human infections by H7N2, H9N2 and H9N9 virus have been reported in the same territory and time frame although people were exposed to these viruses equivalently as to the H7N9 viruses (19), suggesting that these viruses are less-infective to humans than the H7N9 viruses.

These co-circulating viruses were isolated, plaque purified and genotyped. H7N2 and H7N9/Ah was chosen to compare their infectivity in the PD organoids, with the hypothesis that the differentiated airway organoids can indeed simulate human airway epithelium in the context of influenza virus infection. Fig. 4A-C showed that viral loads in the cell lysate and medium of H7N9/Ah-infected organoids gradually increased after inoculation; the viral titer increased more than 3 logio units within 24 hours, indicating a robust viral replication. The addition of serine protease inhibitor AEBSF significantly restricted the active replication of H7N9/Ah virus, highlighting the importance of elevated serine proteases for viral replication. In contrast, H7N2 modestly propagated with viral titer 2~3 logio units lower than H7N9/Ah. Thus, the distinct efficiency of H7N9/Ah and H7N2 to infect and replicate in proximal differentiated airway organoids can recapitulate infectivity of these viruses in humans.

### Establishing 2D airway monolayerfrom airway organoids to assess the infectivity of IA Vs.

3D organoids were transformed into a 2D monolayer using transwell culture. To this end, 3D airway organoids were enzymatically dissociated into single cell suspension, seeded in transwell inserts and then cultured in PD medium. The trans-epithelial electronic resistance (TEER) in the 2D monolayers stabilized in the second week after seeding (Fig. 5A and 5B). In addition, the dextran penetration assay performed at day 12 indicated that an intact epithelial barrier was formed cross the 2D monolayers (Fig. 5A and 5B). The intense signal of ACCTUB indicated that the 2D monolayers developed appreciable proximal differentiation. The productive infection of H7N9/Ah was clearly shown by the virus nucleoprotein (NP) positive cells at 8 hours post infection (hpi).

The replication capacity of H7N9/Ah and H7N2 in the 2D PD organoids was compared. To further verify the ability of 2D PD organoids for assessing zoonotic potential of animal viruses, and identifying the human-infective virus, the replication capacity of H7N9/Ah and H7N2 in the 2D PD organoids, as well as another pair of viruses, the highly human infective HlNlpdm and a swine H1N1 isolate (HlNlsw) were analyzed. The higher replication capacity of H7N9/Ah over H7N2 virus was more pronounced in the 2D PD organoids than in the 3D PD organoids; the viral titer of H7N9/Ah in the apical media was 3~4 logio units higher than that of the H7N2 virus (Figs. 5C and 5D). Consistently, HlNlpdm dramatically replicated with viral titer in apical media 1~2 logio units higher than HlNlsw. Due to the epithelial barrier formed in the 2D monolayers and the preferential virus release from the cell apical side, the viral titers in the basolateral media were consistently lower than those in apical media at the corresponding time points. Nevertheless, the differences in replication capacity between H7N9/Ah versus H7N2, HlNlpdm versus HlNlsw were even more prominent in basal media than in apical media in most time points.

### C. Discussion

This study describes proximal differentiation of human ASC-derived airway organoid culture for studies of a major pathogen, the influenza virus. In particular, the disclosed differentiation conditions increase the numbers of ciliated cells (Figs. 3A and 3C), and the major cell type in the human airway epithelium. The PD medium induces ciliated cell numbers to a near-physiological level, with synchronously beating cilia readily discernible in every organoid. In addition, the expression levels of serine proteases (Fig. 3B) known to be important for productive viral infection, were elevated after proximal differentiation. Among the upregulated HA-activating serine proteases, the dramatically increased expression of HAT in the differentiated airway organoids is very likely attributed to the increased abundance of ciliated cells since ciliated cells are the main source of HAT in the human respiratory tract (Krueger, et al, Swine Influenza Virus Infections in Man. Swine Influenza, eds Richt JA & Webby RJ (Springer Berlin Heidelberg, Berlin, Heidelberg), pp 201-225 (2013)). Thus, the differentiated airway organoids can morphologically and functionally simulate the human airway epithelium. As a further improvement, 2D PD airway monolayers were developed, with an intact epithelial barrier to allow exclusive apical exposure to viruses (Figs. 5A and 5B), the natural mode of IAV infection in the human respiratory tract. Two pairs of viruses with known infectivity were utilized to demonstrate, as a proof-of-concept, that these organoids indeed show significantly higher susceptibility to the human-infective viruses than the poorly human-infective viruses. These 3D and 2D differentiated airway organoids support active replication of human infective H7N9/Ah and HlNlpdm. In contrast, the H7N2 virus, which has been temporally and spatially co-circulating with H7N9 viruses in domestic poultry and contains the similar internal genes as H7N9 viruses, replicated much less efficiently in both models. Similarly, the swine H1N1 isolate showed a lower growth capacity than its counterpart of human-adapted HNlpdm (Figs. 5C and 5D).

The avian IAV H7N2 subtype viruses circulating in the bird market between 1994-2006 caused poultry outbreaks in the US. Sporadic human infections have been reported in the US and Europe. Fortunately, all reported human infection cases experienced mild influenza-like symptoms (Marinova-Petkova, et al, Emerg Infect Dis 23(12) (2017)). While pigs are considered to be the intermediate hosts for interspecies transmission of IAVs, swine influenza viruses lacking human adaptation markers rarely infect humans. Sporadic human infections documented in the literatures or reported by public health officials are generally mild or subclinical (Krueger, et al, Swine Influenza Virus Infections in Man. Swine Influenza, eds Richt JA & Webby RJ (Springer Berlin Heidelberg, Berlin, Heidelberg), pp 201-225 (2013)). The ability of the PD airway organoids to differentiate avian H7 subtype virus and swine Hl subtype virus from the counterpart human viruses suggest that these models could be used for assessment of cross-species transmission potential of emerging influenza virus in humans.

In summary, these differentiated airway organoids significantly extend the current armamentaria of influenza research toolbox.

Unless defined otherwise, all technical and scientific terms used herein have the same meanings as commonly understood by one of skill in the art to which the disclosed invention belongs.

## Claims

1. A method of generating a proximal differentiated airway organoid (PD-organoid) comprising culturing an airway organoid (AO-organoid) derived from adult stem cells (ASC) in a proximal differentiation medium for a period of time sufficient to generate a PD-organoid comprising a cell population consisting of at least 25%, at least 30%, at least 35% or at least 40% ciliated cells, wherein the ciliated cells are **characterised by** FOXJ1 and SNTN expression;
wherein the method further comprises one or more of the following steps prior to culturing the AO-organoid in a proximal differentiation medium:
a. providing a lung tissue sample obtained from a subject;
b. obtaining dissociated cells from a lung tissue sample; and
c. culturing lung cells in an AO-organoid formation phase for a period of time sufficient to generate an AO-organoid; and
wherein the proximal differentiation medium is supplemented with DAPT at a concentration of between 10 and 20 µM.

2. The method of claim 1:
i) wherein the proximal differentiation medium comprises one or more components as set out in Table 2, optionally at the concentrations shown in Table 2;
optionally wherein the proximal differentiation medium comprises at least EGF, insulin, transferrin, hydrocortisone, triiodothyronine and epinephrine further optionally wherein the proximal differentiation medium further comprises bovine serum albumin and/or bovine pituitary extract;
ii) further optionally wherein the proximal differentiation medium is PneumaCult-ALI medium (StemCell Technologies).

3. The method of claim 1, wherein the AO-organoid formation phase comprises culturing cells in an AO-organoid medium comprising one or more components as set out in Table 1, optionally at the concentrations shown in Table 1:
iii) optionally wherein the AO-organoid medium comprises at least R-spondin, a BMP inhibitor, a TGF-beta inhibitor, FGF and heregulin beta-1.

4. The method of any one of claims 1 to 3, wherein the step of culturing the lung cells and/or AO-organoid comprises culturing the cells in contact with an exogenous extracellular matrix (such as a basement membrane extract or MatrigelTM);
optionally wherein the AO-organoid is a 3D organoid, optionally wherein
(i) the PD-organoid is a 3D organoid or
(ii) wherein the PD-organoid is a 2D organoid.

5. The method of claim 4 ii), wherein the step of culturing in a proximal differentiation medium comprises culturing in a transwell culture system comprising an apical and basal chamber.

6. The method of any of claims 1 to 4, wherein the PD-organoid is a 3D PD-organoid in accordance with any one of claims 1 to 4 i) wherein the method comprises:
b. culturing lung cells from a subject in an AO-organoid formation phase in an AO-organoid medium in contact with an extracellular matrix for a period of time sufficient to generate a 3D AO-organoid, for example for at least 2 days; and
c. changing the AO medium to a proximal differentiation medium supplemented with a notch inhibitor and culturing the 3D AO-organoid in the proximal differentiation medium supplemented with a notch inhibitor for a period of time sufficient to generate a PD-organoid, for example for at least 5 days, at least 10 days, at least 14 days or at least 16 days.

7. The method of any of claims 1 to 4, wherein the PD-organoid is a 2D PD-organoid in accordance- with any one of claims 1, 4 ii) or 5 wherein the method comprises the step of:
a. culturing lung cells from a subject in an AO-organoid formation phase in an AO-organoid medium in contact with an extracellular matrix for a period of time sufficient to generate a 3D AO-organoid, for example for at least 2 days;
b. dissociating the 3D AO-organoids into single cell suspension;
c. seeding the dissociated cells in the apical chamber of a transwell culture system;
d. optionally culturing the seeded cells in AO medium for at least 1 day, for example, until the cells reach at least 90% confluence; and
e. culturing the seeded cells in proximal differentiation medium supplemented with a notch inhibitor for a period of time sufficient to generate a 2D PD-organoid, for example for at least 5 days, at least 10 days, at least 14 days or at least 16 days.

8. The method of claim 5 or claim 7, wherein the culture medium is added to both the apical and basal chambers of the transwell culture system;
i) optionally wherein the culture medium is refreshed every other day;
ii) optionally wherein the organoid or cells are human organoids or human cells.

9. A PD-organoid obtained by a method of any one of claims 1 to 8;
i) optionally wherein the PD-organoid has at least 2-fold or at least 3-fold increase in the proportion of ciliated cells when compared to the AO-organoid from which it is derived;
ii) optionally wherein the PD-organoid is further **characterised by** serine protease expression, for example, expression of one or more or all of TMPRSS2, TMPRSS4, TMPRSS11D (HAT) and Matriptase optionally expression of HAT is at least 1 log10 fold increased relative to its expression in AO-organoids;
iii) optionally further comprising one or more or all of the following cell types:
a. basal cells, **characterised by** P63 and CK5 expression;
b. goblet cells, **characterised by** MUC5AC expression; and
c. club cells **characterised by** lack of CC10 and SCGB3A2 expression.

10. The PD-organoid of any one of claims 9, wherein gene expression is assessed using quantitative PCR of mRNA transcripts normalised with GAPDH optionally wherein the PD- organoid further comprises an influenza virus.

11. The method of any one of claims 1 to 8, wherein the PD-organoid has any one or more of the properties described in any one of claims 9 to 10.

12. A method for contracting an influenza virus in a PD-organoid, wherein the method comprises:
a. generating a PD-organoid in accordance with any one of claims 1-11; and
b. infecting the PD-organoid with an influenza virus.

13. The method of claim 12, wherein the infecting step comprises inoculating with the influenza virus at a multiplicity of infection of at least 0.001, at least 0.01or between 0.001 and 0.01;
i) optionally wherein the infecting step further comprises incubating for at least 30 minutes, at least 60 minutes, at least 90 minutes or at least 120 minutes; further optionally wherein the incubating step is performed at about 37°C;
ii) optionally wherein the contacting step is at the apical surface of the PD-organoid;
iii) optionally wherein the PD-organoid is a 2D organoid and the contacting step involves adding the influenza virus to the apical chamber of the transwell culture system.

14. The method of any one of claims 12 to 13, wherein the PD-organoid is a 3D organoid and the method further comprises a step of exposing the apical surface of the 3D organoid, for example by mechanical shearing, prior to contacting the PD-organoid with an influenza virus
i) optionally wherein the method further comprises re-contacting the 3D-organoid with an extracellular matrix and culturing the PD-organoid in a proximal differentiation medium, after infecting, and optionally incubating, the PO-organoid with the influenza virus.

15. The method of any of claims 12 to 14 wherein the method comprises a method for predicting infectivity of the influenza virus to humans and the influenza virus is a test influenza virus, wherein the PD-organoid is a human organoid and wherein the method further comprises:
c. contacting the human PD-organoid with the test influenza virus in accordance with any one of claims 12-14;
d. testing the viral titre after a time period sufficient to allow viral propagation;
e. optionally comparing the viral titre to a control influenza virus;
further optionally wherein testing the viral titre involves detecting a change in viral titre;
i) optionally wherein an increase in viral titre is indicative of likely infectivity of the influenza virus to humans and/or wherein a greater increase over a shorter time period is correlated with a higher degree of infectivity;
ii) optionally wherein the increase in viral titre is at least 1log10 units, at least 2log10 units, or at least 3log10 units within 24 hours.

16. The method of claim 15, wherein the control influenza virus is a known poorly-infective-to-humans influenza virus optionally wherein the change in viral titre of the test influenza virus is greater than the change in viral titre of the known poorly-infective-to-humans influenza virus, for example, wherein the viral titre is at least 10-fold, at least 50-fold, at least 100 fold, at least 1,000 fold or at least 10,000 fold greater than the viral titre of the known poorly-infective-to-humans influenza virus;
i) optionally wherein the known poorly-infective influenza virus is selected from H7N2, H9N2 and H9N9;
or wherein the control influenza virus is a known infective-to-humans influenza virus, optionally wherein the change viral titre of the test influenza virus is about the same or greater than the viral titre of the known infective-to-humans influenza virus, for example, at least 75%, at least 80%, at least 90%, at least 100%, at least 150%, at least 2-fold, at least 5-fold or at least 10-fold relative to the viral titre of the known infective-to-humans influenza virus;
ii) optionally wherein the known infective influenza virus is H7N9.

17. The method or PD-organoid of any one of claims 10-16, wherein the influenza virus is:
a. an influenza A virus;
b. a human, avian or swine influenza virus; and/or
c. an emerging influenza

## Patentansprüche

1. Verfahren zum Erzeugen eines proximalen differenzierten Atemwegsorganoids (PD-Organoid), umfassend Kultivieren eines Atemwegsorganoids (AO-Organoid) abgeleitet von adulten Stammzellen (ASC) in einem proximalen Differenzierungsmedium für einen Zeitraum, der ausreicht, um ein PD-Organoid zu erzeugen, das eine Zellpopulation umfasst, die aus zumindest 25 %, zumindest 30 %, zumindest 35 % oder zumindest 40 % cilierten Zellen besteht, wobei die cilierten Zellen durch FOXJ1- und SNTN-Expression gekennzeichnet sind;
wobei das Verfahren ferner einen oder mehrere der folgenden Schritte vor dem Kultivieren des AO-Organoids in einem proximalen Differenzierungsmedium umfasst:
a. Bereitstellen einer Lungengewebeprobe erhalten von einem Subjekt;
b. Erhalten von dissoziierten Zellen aus einer Lungengewebeprobe; und
c. Kultivieren von Lungenzellen in einer AO-Organoidbildungsphase für einen Zeitraum, der ausreicht, um ein AO-Organoid zu erzeugen; und
wobei das proximale Differenzierungsmedium mit DAPT in einer Konzentration zwischen 10 und 20 µM ergänzt ist.

2. Verfahren nach Anspruch 1:
i) wobei das proximale Differenzierungsmedium eine oder mehrere Komponenten wie dargelegt in Tabelle 2 umfasst, optional in den Konzentrationen, die in Tabelle 2 gezeigt sind;
wobei optional das proximale Differenzierungsmedium zumindest EGF, Insulin, Transferrin, Hydrocortison, Triiodthyronin und Epinephrin umfasst, wobei ferner optional das proximale Differenzierungsmedium ferner Rinderserumalbumin und/oder Rinderhypophysenextrakt umfasst;
ii) wobei ferner optional das proximale Differenzierungsmedium PneumaCult-ALI-Medium (StemCell Technologies) ist.

3. Verfahren nach Anspruch 1, wobei die AO-Organoidbildungsphase Kultivieren von Zellen in einem AO-Organoidmedium umfasst, das eine oder mehrere Komponenten wie dargelegt in Tabelle 1 umfasst, optional in den Konzentrationen, die in Tabelle 1 gezeigt sind:
iii) wobei optional das AO-Organoidmedium zumindest R-Spondin, einen BMP-Hemmer, einen TGF-Beta-Hemmer, FGF und Heregulin beta-1 umfasst.

4. Verfahren nach einem der Ansprüche 1 bis 3, wobei der Schritt des Kultivierens der Lungenzellen und/oder des AO-Organoids Kultivieren der Zellen in Kontakt mit einer exogenen extrazellulären Matrix (wie einem Basalmembranextrakt oder MatrigelTM) umfasst;
wobei optional das AO-Organoid ein 3D-Organoid ist, wobei optional
(i) das PD-Organoid ein 3D-Organoid ist, oder
(ii) wobei das PD-Organoid ein 2D-Organoid ist.

5. Verfahren nach Anspruch 4 ii), wobei der Schritt des Kultivierens in einem proximalen Differenzierungsmedium Kultivieren in einem Transwell-Kultursystem umfasst, das eine apikale und basale Kammer umfasst.

6. Verfahren nach einem der Ansprüche 1 bis 4, wobei das PD-Organoid ein 3D-PD-Organoid nach einem der Ansprüche 1 bis 4 i) ist, wobei das Verfahren Folgendes umfasst:
b. Kultivieren von Lungenzellen von einem Subjekt in einer AO-Organoidbildungsphase in einem AO-Organoidmedium in Kontakt mit einer extrazellulären Matrix für einen Zeitraum, der ausreicht, um ein 3D-AO-Organoid zu erzeugen, zum Beispiel für zumindest 2 Tage; und
c. Ändern des AO-Mediums zu einem proximalen Differenzierungsmedium, das mit einem Notch-Hemmer ergänzt ist, und Kultivieren des 3D-AO-Organoids in dem proximalen Differenzierungsmedium, das mit einem Notch-Inhibitor ergänzt ist, für einen Zeitraum, der ausreicht, um ein PD-Organoid zu erzeugen, zum Beispiel für zumindest 5 Tage, zumindest 10 Tage, zumindest 14 Tage oder zumindest 16 Tage.

7. Verfahren nach einem der Ansprüche 1 bis 4, wobei das PD-Organoid ein 2D-PD-Organoid nach einem der Ansprüche 1, 4 ii) oder 5 ist, wobei das Verfahren den folgenden Schritt umfasst:
a. Kultivieren von Lungenzellen von einem Subjekt in einer AO-Organoidbildungsphase in einem AO-Organoidmedium in Kontakt mit einer extrazellulären Matrix für einen Zeitraum, der ausreicht, um ein 3D-AO-Organoid zu erzeugen, zum Beispiel für zumindest 2 Tage;
b. Dissoziieren der 3D-AO-Organoide in Einzelzellsuspension;
c. Säen der dissoziierten Zellen in die apikale Kammer eines Transwell-Kultursystems;
d. optional Kultivieren der gesäten Zellen in AO-Medium für zumindest 1 Tag, zum Beispiel, bis die Zellen zumindest 90 % Konfluenz erreichen; und
e. Kultivieren der gesäten Zellen in proximalem Differenzierungsmedium, das mit einem Notch-Hemmer ergänzt ist, für einen Zeitraum, der ausreicht, um ein 2D-PD-Organoid zu erzeugen, zum Beispiel für zumindest 5 Tage, zumindest 10 Tage, zumindest 14 Tage oder zumindest 16 Tage.

8. Verfahren nach Anspruch 5 oder Anspruch 7, wobei das Kulturmedium sowohl der apikalen als auch der basalen Kammer des Transwell-Kultursystems hinzugefügt wird;
i) wobei optional das Kulturmedium jeden zweiten Tag aufgefrischt wird;
ii) wobei optional das Organoid oder die Zellen menschliche Organoide oder menschliche Zellen sind.

9. PD-Organoid, das durch ein Verfahren nach einem der Ansprüche 1 bis 8 erhalten wird;
i) wobei optional das PD-Organoid zumindest 2-fache oder zumindest 3-fache Erhöhung des Anteils an ciliierten Zellen im Vergleich zu dem AO-Organoid, von dem es abgeleitet ist, aufweist;
ii) wobei optional das PD-Organoid ferner durch Serinprotease-Expression gekennzeichnet ist, zum Beispiel Expression von einem oder mehreren oder allen von TMPRSS2, TMPRSS4, TMPRSS11D (HAT) und Matriptase, optional Expression von HAT zumindest 1 log 10-fach relativ zu seiner Expression in AO-Organoiden erhöht ist;
iii) optional ferner umfassend einen oder mehrere oder alle der folgenden Zelltypen:
a. Basalzellen, **gekennzeichnet durch** P63- und CK5-Expression;
b. Becherzellen, **gekennzeichnet durch** MUC5AC-Expression; und
c. Clubzellen, **gekennzeichnet durch** Fehlen von CC10- und SCGB3A2-Expression.

10. PD-Organoid nach einem der Ansprüche 9, wobei Genexpression unter Verwendung von quantitativer PCR von mRNA-Transkripten, die mit GAPDH normalisiert sind, beurteilt wird, wobei optional das PD-Organoid ferner ein Influenzavirus umfasst.

11. Verfahren nach einem der Ansprüche 1 bis 8, wobei das PD-Organoid eine oder mehrere der Eigenschaften beschrieben in einem der Ansprüche 9 bis 10 aufweist.

12. Verfahren zum Kontrahieren eines Influenzavirus in einem PD-Organoid, wobei das Verfahren Folgendes umfasst:
a. Erzeugen eines PD-Organoids nach einem der Ansprüche 1-11; und
b. Infizieren des PD-Organoids mit einem Influenzavirus.

13. Verfahren nach Anspruch 12, wobei der Infektionsschritt Impfen mit dem Influenzavirus bei einer Mehrzahl von Infektionen von zumindest 0,001, zumindest 0,01 oder zwischen 0,001 und 0,01 umfasst;
i) wobei optional der Infektionsschritt ferner Inkubieren für zumindest 30 Minuten, zumindest 60 Minuten, zumindest 90 Minuten oder zumindest 120 Minuten umfasst; wobei ferner optional der Inkubationsschritt bei etwa 37 °C durchgeführt wird;
ii) wobei optional der Kontaktierungsschritt an der apikalen Oberfläche des PD-Organoids ist;
iii) wobei optional das PD-Organoid ein 2D-Organoid ist und der Kontaktierungsschritt Hinzufügen des Influenzavirus zu der apikalen Kammer des Transwell-Kultursystems involviert.

14. Verfahren nach einem der Ansprüche 12 bis 13, wobei das PD-Organoid ein 3D-Organoid ist und das Verfahren ferner einen Schritt des Freilegens der apikalen Oberfläche des 3D-Organoids, zum Beispiel durch mechanisches Scheren, vor dem Kontaktieren des PD-Organoids mit einem Influenzavirus umfasst,
i) wobei optional das Verfahren ferner erneutes Kontaktieren des 3D-Organoids mit einer extrazellulären Matrix und Kultivieren des PD-Organoids in einem proximalen Differenzierungsmedium nach dem Infizieren und optional Inkubieren des PO-Organoids mit dem Influenzavirus umfasst.

15. Verfahren nach einem der Ansprüche 12 bis 14, wobei das Verfahren ein Verfahren zum Vorhersagen von Infektiosität des Influenzavirus für Menschen umfasst und das Influenzavirus ein Test-Influenzavirus ist, wobei das PD-Organoid ein menschliches Organoid ist und wobei das Verfahren ferner Folgendes umfasst:
c. Kontaktieren des menschlichen PD-Organoids mit dem Test-Influenzavirus nach einem der Ansprüche 12-14;
d. Testen des Virustiters nach einem Zeitraum, der ausreicht, um Virusvermehrung zu ermöglichen;
e. optional Vergleichen des Virustiters mit einem Kontroll-Influenzavirus;
wobei ferner optional das Testen des Virustiters Detektieren einer Änderung des Virustiters involviert;
i) wobei optional eine Erhöhung des Virustiters auf wahrscheinliche Infektiosität des Influenzavirus für Menschen hinweist und/oder wobei eine größere Erhöhung über einen kürzeren Zeitraum mit einem höheren Grad an Infektiosität korreliert;
ii) wobei optional die Erhöhung des Virustiters zumindest 1log10 Einheiten, zumindest 2log10 Einheiten oder zumindest 3log10 Einheiten innerhalb von 24 Stunden ist.

16. Verfahren nach Anspruch 15, wobei das Kontroll-Influenzavirus ein bekanntes Influenzavirus mit geringer Infektiosität für Menschen ist, wobei optional die Änderung des Virustiters des Test-Influenzavirus größer als die Änderung des Virustiters des bekannten Influenzavirus mit geringer Infektiosität für Menschen ist, wobei zum Beispiel der Virustiter zumindest 10-fach, zumindest 50-fach, zumindest 100-fach, zumindest 1.000-fach oder zumindest 10.000-fach größer als der Virustiter des bekannten Influenzavirus mit geringer Infektiosität für Menschen ist;
i) wobei optional das bekannte Influenzavirus mit geringer Infektiosität aus H7N2, H9N2 und H9N9 ausgewählt ist;
oder wobei das Kontroll-Influenzavirus ein bekanntes Influenzavirus mit Infektiosität für Menschen ist, wobei optional die Änderung des Virustiters des Test-Influenzavirus etwa gleich dem oder größer als der Virustiter des bekannten Influenzavirus mit Infektiosität für Menschen ist, zum Beispiel zumindest 75 %, zumindest 80 %, zumindest 90 %, zumindest 100 %, zumindest 150 %, zumindest 2-fach, zumindest 5-fach oder zumindest 10-fach relativ zu dem Virustiter des bekannten Influenzavirus mit Infektiosität für Menschen;
ii) wobei optional das bekannte infektiöse Influenzavirus H7N9 ist.

17. Verfahren oder PD-Organoid nach einem der Ansprüche 10-16, wobei das Influenzavirus wie folgt ist:
a. ein Influenza-A-Virus;
b. ein Influenzavirus bei Menschen, Geflügel oder Schwein; und/oder
c. eine neu auftretende Influenza.

## Revendications

1. Procédé de génération d'un organoïde différencié proximal (organoïde-DP) de voies aériennes comprenant la culture d'un organoïde de voies aériennes (organoïde-VA) dérivé de cellules souches adultes (ASC) dans un milieu de différenciation proximal pendant une période de temps suffisante pour générer un organoïde-DP comprenant une population cellulaire consistant d'au moins 25 %, d'au moins 30 %, d'au moins 35 % ou d'au moins 40 % de cellules ciliées, lesdites cellules ciliées étant **caractérisées par** l'expression de FOXJ1 et de SNTN ;
ledit procédé comprenant en outre une ou plusieurs des étapes suivantes avant de cultiver l'organoïde-VA dans un milieu de différenciation proximal :
a. la fourniture d'un échantillon de tissu pulmonaire prélevé chez un sujet ;
b. l'obtention de cellules dissociées à partir d'un échantillon de tissu pulmonaire ; et
c. la culture de cellules pulmonaires dans une phase de formation d'organoïde-VA pendant une période de temps suffisante pour générer un organoïde-VA ; et
ledit milieu de différenciation proximal étant complété par du DAPT à une concentration située entre 10 et 20 µM.

2. Procédé selon la revendication 1 :
i) ledit milieu de différenciation proximal comprenant un ou plusieurs composants comme indiqué dans le tableau 2, éventuellement aux concentrations indiquées dans le tableau 2 ;
éventuellement ledit milieu de différenciation proximal comprenant au moins de l'EGF, de l'insuline, de la transferrine, de l'hydrocortisone, de la triiodothyronine et de l'épinéphrine, en outre éventuellement ledit milieu de différenciation proximal comprenant en outre de l'albumine sérique bovine et/ou un extrait hypophysaire bovin ;
ii) éventuellement, ledit milieu de différenciation proximal étant le milieu PneumaCult-ALI (StemCell Technologies).

3. Procédé selon la revendication 1, ladite phase de formation d'organoïde-VA comprenant la culture de cellules dans un milieu d'organoïde-VA comprenant un ou plusieurs composants comme indiqué dans le tableau 1, éventuellement aux concentrations indiquées dans le tableau 1 :
iii) éventuellement ledit milieu d'organoïde-VA comprenant au moins de la R-spondine, un inhibiteur de BMP, un inhibiteur de TGF-bêta, du FGF et de l'héréguline bêta-1.

4. Procédé selon l'une quelconque des revendications 1 à 3, ladite étape de culture des cellules pulmonaires et/ou de l'organoïde-VA comprenant la culture des cellules en contact avec une matrice extracellulaire exogène (telle qu'un extrait de membrane basale ou le MatrigelTM) ;
éventuellement ledit organoïde-VA étant un organoïde 3D, éventuellement
(i) ledit organoïde-DP étant un organoïde 3D ou
(ii) ledit organoïde-DP étant un organoïde 2D.

5. Procédé selon la revendication 4 ii), ladite étape de culture dans un milieu de différenciation proximal comprenant la culture dans un système de culture transwell comprenant une chambre apicale et une chambre basale.

6. Procédé selon l'une quelconque des revendications 1 à 4, ledit organoïde-DP étant un organoïde-DP 3D selon l'une quelconque des revendications 1 à 4 i), ledit procédé comprenant :
b. la culture de cellules pulmonaires provenant d'un sujet dans une phase de formation d'organoïde-VA dans un milieu d'organoïde-VA en contact avec une matrice extracellulaire pendant une période de temps suffisante pour générer un organoïde-VA 3D, par exemple pendant au moins 2 jours ; et
c. le changement du milieu VA en un milieu de différenciation proximal complété par un inhibiteur de Notch et la culture de l'organoïde-VA 3D dans le milieu de différenciation proximal complété par un inhibiteur de Notch pendant une période de temps suffisante pour générer un organoïde-DP, par exemple pendant au moins 5 jours, au moins 10 jours, au moins 14 jours ou au moins 16 jours.

7. Procédé selon l'une quelconque des revendications 1 à 4, ledit organoïde-DP étant un organoïde-DP 2D selon l'une quelconque des revendications 1, 4 ii) ou 5, ledit procédé comprenant l'étape de :
a. culture de cellules pulmonaires provenant d'un sujet dans une phase de formation d'organoïde-VA dans un milieu d'organoïde-VA en contact avec une matrice extracellulaire pendant une période de temps suffisante pour générer un organoïde-VA 3D, par exemple pendant au moins 2 jours ;
b. dissociation des organoïdes-VA 3D en suspension unicellulaire ;
c. ensemencement des cellules dissociées dans la chambre apicale d'un système de culture transwell ;
d. culture éventuelle des cellules ensemencées dans un milieu VA pendant au moins 1 jour, par exemple, jusqu'à ce que les cellules atteignent une confluence d'au moins 90 % ; et
e. culture des cellules ensemencées dans un milieu de différenciation proximal complété par un inhibiteur de Notch pendant une période de temps suffisante pour générer un organoïde-DP 2D, par exemple pendant au moins 5 jours, au moins 10 jours, au moins 14 jours ou au moins 16 jours.

8. Procédé selon la revendication 5 ou la revendication 7, ledit milieu de culture étant ajouté à la fois aux chambres apicale et basale du système de culture transwell ;
i) éventuellement ledit milieu de culture étant rafraîchi tous les deux jours ;
ii) éventuellement, ledit organoïde ou lesdites cellules étant des organoïdes humains ou des cellules humaines.

9. Organoïde-DP obtenu par un procédé selon l'une quelconque des revendications 1 à 8 ;
i) éventuellement ledit organoïde-DP présentant une augmentation d'un facteur d'au moins 2 ou d'au moins 3 de la proportion de cellules ciliées par rapport à l'organoïde-VA dont il est dérivé ;
ii) éventuellement, ledit organoïde-DP étant en outre **caractérisé par** l'expression de la sérine protéase, par exemple, l'expression d'un ou plusieurs ou tous les éléments parmi TMPRSS2, TMPRSS4, TMPRSS11D (HAT) et ladite expression éventuelle de la matriptase de HAT étant augmentée d'un facteur d'au moins 1 log10 par rapport à son expression dans les organoïdes-VA ;
iii) comprenant éventuellement en outre un ou plusieurs ou tous les types de cellules suivants :
a. les cellules basales, **caractérisées par** l'expression de P63 et de CK5 ;
b. les cellules calciformes, **caractérisées par** l'expression de MUC5AC ; et
c. les cellules clubs **caractérisées par** un manque d'expression de CC10 et de SCGB3A2.

10. Organoïde-DP selon l'une quelconque des revendications 9, ladite expression génique étant évaluée en utilisant une PCR quantitative de transcrits d'ARNm normalisés avec GAPDH éventuellement ledit organoïde-DP comprenant en outre un virus de la grippe.

11. Procédé selon l'une quelconque des revendications 1 à 8, ledit organoïde-DP présentant l'une quelconque ou plusieurs des propriétés décrites dans l'une quelconque des revendications 9 à 10.

12. Procédé permettant de contracter un virus de la grippe dans un organoïde-DP, ledit procédé comprenant :
a. la génération d'un organoïde-DP selon l'une quelconque des revendications 1 à 11 ; et
b. l'infection de l'organoïde-DP avec un virus de la grippe.

13. Procédé selon la revendication 12, ladite étape d'infection comprenant l'inoculation avec le virus de la grippe à une multiplicité d'infection d'au moins 0,001 d'au moins 0,01 ou située entre 0,001 et 0,01 ;
i) éventuellement ladite étape d'infection comprenant en outre l'incubation pendant au moins 30 minutes, au moins 60 minutes, au moins 90 minutes ou au moins 120 minutes ; en outre éventuellement ladite étape d'incubation étant effectuée à environ 37°C ;
ii) éventuellement ladite étape de mise en contact se trouvant au niveau de la surface apicale de l'organoïde-DP ;
iii) éventuellement, ledit organoïde-DP étant un organoïde 2D et ladite étape de mise en contact impliquant l'ajout du virus de la grippe à la chambre apicale du système de culture transwell.

14. Procédé selon l'une quelconque des revendications 12 à 13, ledit organoïde-DP étant un organoïde 3D et ledit procédé comprenant en outre une étape d'exposition de la surface apicale de l'organoïde 3D, par exemple par cisaillement mécanique, avant la mise en contact de l'organoïde-DP avec un virus de la grippe
i) éventuellement, ledit procédé comprenant en outre la remise en contact de l'organoïde 3D avec une matrice extracellulaire et la culture de l'organoïde-DP dans un milieu de différenciation proximal, après infection, et éventuellement l'incubation de l'organoïde-PO avec le virus de la grippe.

15. Procédé selon l'une quelconque des revendications 12 à 14, ledit procédé comprenant un procédé permettant de prédire l'infectivité du virus de la grippe pour l'homme et ledit virus de la grippe étant un virus de la grippe test, ledit organoïde-DP étant un organoïde humain et ledit procédé comprenant en outre :
c. la mise en contact de l'organoïde-DP humain avec le virus de la grippe test selon l'une quelconque des revendications 12 à 14 ;
d. le test du titre viral après une période de temps suffisante pour permettre la propagation virale ;
e. la comparaison éventuelle du titre viral à un virus de la grippe témoin ;
en outre, éventuellement, ledit test du titre viral impliquant la détection d'un changement du titre viral ;
i) éventuellement une augmentation du titre viral indiquant une infectivité probable du virus de la grippe pour l'homme et/ou une augmentation plus importante sur une période de temps plus courte étant corrélée à un degré d'infectivité plus élevé ;
ii) éventuellement ladite augmentation du titre viral étant d'au moins 1 log10 unités, d'au moins 2 log10 unités ou d'au moins 3 log10 unités dans un délai de 24 heures.

16. Procédé selon la revendication 15, ledit virus de la grippe témoin étant un virus de la grippe faiblement infectieux pour les humains connu, éventuellement ledit changement du titre viral du virus de la grippe test étant supérieur au changement du titre viral du virus de la grippe faiblement infectieux pour les humains connu, par exemple, ledit titre viral étant au moins 10 fois, au moins 50 fois, au moins 100 fois, au moins 1 000 fois ou au moins 10 000 fois supérieur au titre viral du virus de la grippe faiblement infectieux pour les humains connu ;
i) éventuellement ledit virus de la grippe faiblement infectieux connu étant choisi parmi H7N2, H9N2 et H9N9 ;
ou ledit virus de la grippe témoin étant un virus de la grippe infectieux pour les humains connu, éventuellement ledit changement de titre viral du virus de la grippe test étant à peu près supérieur ou égal au titre viral du virus de la grippe infectieux pour les humains connu, par exemple, au moins 75 %, au moins 80 %, au moins 90 %, au moins 100 %, au moins 150 %, d'un facteur d'au moins 2, d'un facteur d'au moins 5 ou d'un facteur d'au moins 10 par rapport au titre viral du virus de la grippe infectieux pour les humains connu ;
ii) éventuellement ledit virus de la grippe infectieux connu étant H7N9.

17. Procédé ou organoïde-DP selon l'une quelconque des revendications 10 à 16, ledit virus de la grippe étant :
a. un virus de la grippe A ;
b. un virus de la grippe humaine, aviaire ou porcine ; et/ou
c. une grippe émergente.
